# EUROPEAN PATENT APPLICATION

(11) **EP 1 726 251 A1**
(43) Date of publication of application: **29.11.2006**
(21) Application number: 05726715.5
(22) Date of filing: 18.03.2005
(51) Int. Cl.: A61B 1/00

(54) **INSERTION DEVICE**

(30) Priority: 18.03.2004 JP 2004079308; 19.03.2004 JP 2004081651; 19.03.2004 JP 2004081652; 19.03.2004 JP 2004081656
(71) Applicant: Olympus Corporation, Tokyo 151-0072 (JP)
(72) Inventor: KURA, Yasuhito c/o Olympus Intellectual Property, Tokyo 192-8512 (JP); ADACHI,Katsutaka c/o Olympus Intellectual Property, Tokyo 192-8512 (JP)
(74) Representative: von Hellfeld, Axel
(86) International application number: PCT/JP2005/004996
(87) International publication number: WO 2005/089630

(57) **Abstract**

An insertion device comprises an insertion unit for assisting insertion into a subject of medical equipment, which is inserted into a subject for performing medical activity, the insertion unit being capable of advancing or retreating relatively as to the medical equipment in the insertion direction into the subject, and a rotating unit for rotating the insertion unit centered on the insertion axial direction, and generating propulsion as to insertion of the insertion unit into the subject.

## Description

### Technical Field

The present invention relates to an insertion device for improving ease of insertion of medical equipment into a body cavity.

### Background Art

Heretofore, endoscopes for observing an organ within a lumen by inserting a slender insertion unit included therein into the lumen, inserting a treatment tool into a treatment insertion channel provided in the insertion unit as necessary, so as to be capable of various types of therapy and treatment, have been widely employed.

In general, endoscopes including a slender insertion unit are provided with a bending portion at the tip side of the insertion unit. The bending portion performs a bending operation, for example, in the vertical and horizontal directions by an operating wire connected to a bending piece making up the bending portion being advanced or retreated. Advancing and retreating of the operating wire can be performed by a surgeon moving rotationally, for example, a bending knob provided in an operating unit.

When inserting the insertion unit into an intricate lumen, e.g., a lumen such as the large intestine which follows a loop of 360 degrees, the surgeon operates the bending knob to subject the bending portion to a bending operation, and also twists and operates the insertion unit to insert the tip portion of the insertion unit toward an observation target portion.

However, it requires a seasoned surgeon to be able to insert the insertion unit smoothly into the intricate large intestine in a short period of time without causing the patient pain. In the case of an inexperienced surgeon, the surgeon has been at risk of mistaking the insertion direction and taking time in insertion when inserting the insertion unit into a deep portion, or at risk of deforming the course of the intestine when inserting the insertion unit toward a deep portion. Accordingly, various types of proposals have been made for improving ease of insertion of an insertion unit.

For example, Japanese Unexamined Patent Application Publication No. 10-113396 has disclosed a propulsion device for medial equipment capable of readily leading the medical equipment to a deep portion of a living-body tube with low invasiveness. With this propulsion device, a rotating member is provided with diagonal ribs as to the axial direction of this rotating member. Accordingly, the rotating force of the rotating member is converted by the ribs into propulsion by subjecting the rotating member to a rotational operation, and the medical equipment connected to the propulsion device is moved in a deep portion direction by the propulsion.

However, with the propulsion device for medical equipment according to Japanese Unexamined Patent Application Publication No. 10-113396, the rotating member serving as an insertion assisting member can rotate at the tip side of medical equipment as to the medical equipment, but is configured so as to be provided generally integral with the medical equipment. Accordingly, in the event that at least either of, for example, observation means for observing the inside of a subject, or the opening of a treatment-tool insertion channel for inserting a treatment tool for performing treatment within a subject is provided at the tip of the medical equipment, applying the above technology to the medical equipment, causes the following problems. When observing a subject, the surgeon is at risk of the rotating member disturbing the observation visual field of the observation means. In the event of performing treatment as to a treatment target portion of a subject, the surgeon is at risk of the rotating member disturbing the treatment being performed by the surgeon.

The present invention has been made in light of the above situations, and it is an object thereof to provide an insertion device for improving ease of insertion of medical equipment into a body cavity without deteriorating the functions which the medical equipment has.

### Disclosure of Invention

### Means for Solving the Problems

According to an insertion device of the present invention, the insertion device comprises an insertion unit for assisting insertion into a subject of medical equipment, which is inserted into a subject for performing medical activity, the insertion unit being capable of advancing or retreating relatively as to the medical equipment in the insertion direction into the subject, and a rotating unit for rotating the insertion unit centered on the insertion axial direction, and generating propulsion at the time of inserting the insertion unit into the subject.

### Brief Description of the Drawings

Fig. 1 is a diagram describing the configuration of an insertion device.
Fig. 2 is a diagram describing the configuration of an insertion-unit guide member.
Fig. 3 is a diagram describing an insertion state of the insertion-unit guide member into the large intestine.
Fig. 4 is a diagram illustrating the insertion-unit guide member inserted near the appendix.
Fig. 5 is a diagram describing a procedure for inserting the insertion-unit guide member into a treatment-tool insertion channel provided in the insertion unit of an endoscope.
Fig. 6 is a diagram describing a state in which the insertion unit of the endoscope is inserted into the large intestine with the insertion-unit guide member serving as a guide.
Fig. 7 is a diagram describing another configuration of the insertion-unit guide member.
Fig. 8 is a diagram describing yet another configuration of the insertion-unit guide member.
Fig. 9 is a diagram describing a configuration example of the insertion-unit guide member including a first guide tube portion and a second guide tube portion.
Fig. 10 is a diagram illustrating a metal wire configuring the first guide tube portion and the second guide tube portion.
Fig. 11 is a cross-sectional view taken along the line XI-XI in Fig. 10.
Fig. 12 is a cross-sectional view taken along the line XII-XII in Fig. 10.
Fig. 13 is a diagram describing another configuration example of the insertion-unit guide member including the first guide tube portion and the second guide tube portion.
Fig. 14 is a diagram describing yet another configuration example of the insertion-unit guide member including the first guide tube portion and the second guide tube portion.
Fig. 15 is a diagram describing another configuration of the insertion-unit guide member.
Fig. 16 is a diagram describing yet another configuration of the insertion-unit guide member.
Fig. 17 is a diagram the configuration of an insertion-unit guide tube.
Fig. 18 is a diagram illustrating a state in which the guider of the insertion-unit guide tube has reached a sigmoid colon portion.
Fig. 19 is a diagram illustrating a state in which the guider of the insertion-unit guide tube has passed over a fold of the intestinal wall of the sigmoid colon portion.
Fig. 20 is a diagram illustrating a state in which the guider of the insertion-unit guide tube has passed through the sigmoid colon portion.
Fig. 21 is a diagram illustrating the entire large intestine illustrating the flexible state of the insertion-unit guide tube, the guider within the large intestine, and a wire member.
Fig. 22 is a diagram illustrating the flexible state of the insertion-unit guide tube in a state in which the guider has reached near the appendix.
Fig. 23 is a diagram describing the configuration of the guider including a through hole through which a wire shaft passes.
Fig. 24 is a diagram describing the configuration of a wire shaft including a different eccentric axis.
Fig. 25 is a transverse cross-sectional view of the insertion-unit guide tube.
Fig. 26 is a front view in which the insertion-unit guide tube is viewed from the halfway through the wire shaft.
Fig. 27 is a diagram illustrating a state in which the guider of the insertion-unit guide tube has reached a bending portion of the large intestine.
Fig. 28 is a diagram illustrating a state in which the insertion-unit guide tube rotates.
Fig. 29 is a diagram describing the configuration of an insertion-unit guide tube.
Fig. 30 is a diagram illustrating a state in which the guider of the insertion-unit guide tube has reached a bending portion of the large intestine.
Fig. 31 is a diagram describing a state in which the guider of the insertion-unit guide tube has passed over a fold of the intestinal wall of the large intestine.
Fig. 32 is a diagram describing the configuration of an insertion-unit guide tube.
Fig. 33 is a diagram describing the configuration of an insertion-unit guide tube.
Fig. 34 is a diagram describing the configuration of an insertion-unit guide tube in which spheres and a holding member are provided.
Fig. 35 is a cross-sectional view of the tip portion of an insertion-unit guide tube in which spheres and a holding member are provided.
Fig. 36 is a diagram describing the configuration of an insertion-unit guide tube in which multiple rotators are provided.
Fig. 37 is a principal-portion enlarged view enlarging and illustrating the principal portions of the insertion-unit guide tube.
Fig. 38 is a diagram illustrating a state in which the tip guide member of the insertion-unit guide tube is in contact with the wall face of a bending portion of the sigmoid colon portion.
Fig. 39 is a diagram illustrating a state in which the insertion-unit guide tube has further advanced, and a part of the tip guide member is bent.
Fig. 40 is a diagram illustrating a state in which the insertion-unit guide tube has further advanced, and a part of the tip guide member is further bent.
Fig. 41 is a diagram illustrating a state in which the insertion-unit guide tube has been inserted into near the appendix.
Fig. 42 is a principal-portion enlarged view enlarging and illustrating a part of the configuration of the insertion-unit guide tube.
Fig. 43 is a diagram illustrating an operation at the time of inserting the insertion-unit guide tube into the large intestine, and illustrating a state in which the tip guide member of the insertion-unit guide tube has come into contact with the wall face a bending portion of the sigmoid colon portion.
Fig. 44 is a diagram illustrating an operation at the time of inserting the insertion-unit guide tube into the large intestine, and illustrating a state in which a part of the tip guide member is bent by further pushing the insertion-unit guide tube forward.
Fig. 45 is a diagram illustrating an operation at the time of inserting the insertion-unit guide tube into the large intestine, and illustrating a state in which a part of the tip guide member is further bent by further pushing the insertion-unit guide tube forward.
Fig. 46 is a principal-portion enlarged view enlarging and illustrating a part of the configuration of an endoscope insertion assisting tool to be employed for an insertion device.
Fig. 47 is a principal-portion enlarged view enlarging and illustrating a part of the configuration of an endoscope insertion assisting tool to be employed for an insertion device.
Fig. 48 is a diagram illustrating an operation at the time of inserting the insertion-unit guide tube into the large intestine, and illustrating a state in which the tip guide member of the insertion-unit guide tube has come into contact with the wall face of a bending portion of the sigmoid colon portion.
Fig. 49 is a diagram illustrating an operation at the time of inserting the insertion-unit guide tube into the large intestine, and illustrating a state in which a part of the tip guide member is bent by further pushing the insertion-unit guide tube forward.
Fig. 50 is a diagram illustrating operations at the time of inserting the insertion-unit guide tube into the large intestine, and illustrating a state in which a part of the tip guide member is further bent by pushing in the insertion-unit guide tube.
Fig. 51 is a diagram describing the configuration of an insertion-unit guide tube in which a capsule endoscope is provided.
Fig. 52 is a diagram illustrating a state in which the capsule-type endoscope of the insertion-unit guide tube has reached a bending portion of the large intestine.
Fig. 53 is a diagram illustrating a state in which the capsule-type endoscope of the insertion-unit guide tube has passed over a fold of the intestinal wall of the large intestine.
Fig. 54 is a diagram describing the configuration of an insertion-unit guide tube in which a capsule endoscope is provided.
Fig. 55 is a cross-sectional view of the tip portion of the insertion-unit guide tube in which the capsule endoscope shown in Fig. 54 is provided.
Fig. 56 is a diagram describing the configuration of an insertion-unit guide tube in which a capsule endoscope is provided.
Fig. 57 is a diagram describing the configuration of an insertion-unit guide tube in which a capsule endoscope is provided.
Fig. 58 is a transverse cross-sectional view of the tip portion of the insertion-unit guide tube in which the capsule endoscope shown in Fig. 57 is provided.
Fig. 59 is a transverse cross-sectional view of an insertion-unit guide tube in which a balloon is provided.
Fig. 60 is a diagram illustrating a state in which the insertion-unit guide tube in which a balloon is provided has reached a bending portion of the large intestine.
Fig. 61 is a diagram describing distention of the balloon of the insertion-unit guide tube inserted into the large intestine.
Fig. 62 is a longitudinal-direction cross-sectional view describing the configuration of a guide-member rotating device.
Fig. 63 is a front view describing the configuration of the guide-member rotating device.
Fig. 64 is a longitudinal-direction cross-sectional view describing a guide-tube rotating device which rotates a guide tube, and also is subjected to straight-ahead movement.
Fig. 65 is a front view describing the guide-tube rotating device.
Fig. 66 is a diagram describing an endoscope mounting a guide-tube insertion assisting tool at the insertion unit thereof, and an insertion-unit guide member.
Fig. 67 is a diagram describing an endoscope mounting a tip cap at the insertion unit thereof, and an insertion-unit guide member.
Fig. 68 is a diagram describing an endoscope in which is provided a guide tube insertion salient portion forming a guide-tube insertion hole at the insertion unit thereof, and an insertion-unit guide member.
Fig. 69 is a diagram describing the insertion unit of an endoscope which is inserted while capturing an insertion-unit guide member.
Fig. 70 is a diagram describing another configuration example of an insertion device.
Fig. 71 is a diagram describing the configuration of an insertion device.
Fig. 72 is a diagram describing the configuration of an insertion-unit guide member, and also describing the positional relation between the insertion unit of an endoscope and an insertion-unit guide member.
Fig. 73 is a diagram describing the insertion-unit guide member disposed at the outer circumferential side of the insertion unit disposed within the large intestine.
Fig. 74 is a diagram describing the insertion-unit guide member disposed at the outer circumferential side of the insertion unit which has reached near the appendix of the large intestine.
Fig. 75 is a diagram describing the configuration of an insertion-unit guide member in which positioning rotational moving means is provided, and an insertion unit.
Fig. 76 is a diagram describing another positional relation between the insertion unit of an endoscope and an insertion-unit guide member.
Fig. 77 is a diagram describing a state in which the insertion unit is inserted into the large intestine, while the bending portion of the insertion unit protruding from the insertion-unit guide member.
Fig. 78 is a diagram describing a state in which the insertion unit is further inserted into a deep portion of the large intestine, while the bending portion of the insertion unit protruding from the insertion-unit guide member .
Fig. 79 is a diagram describing the configuration of an insertion-unit guide member.
Fig. 80 is a diagram describing a state in which the insertion-unit guide member is inserted into the large intestine using a guide-member rotating device.
Fig. 81 is a diagram describing a state in which the insertion-unit guide member is inserted from the anus.
Fig. 82 is a diagram describing a state in which the insertion-unit guide member is inserted into a deep portion of the large intestine.
Fig. 83 is a diagram describing a state in which the insertion unit of the endoscope is inserted into near the appendix via an internal hole of the insertion-unit guide member inserted into the deep portion of the large intestine.

### Best Mode for Carrying Out the Invention

Hereinafter, embodiments of the present invention will be described with reference to the drawings.

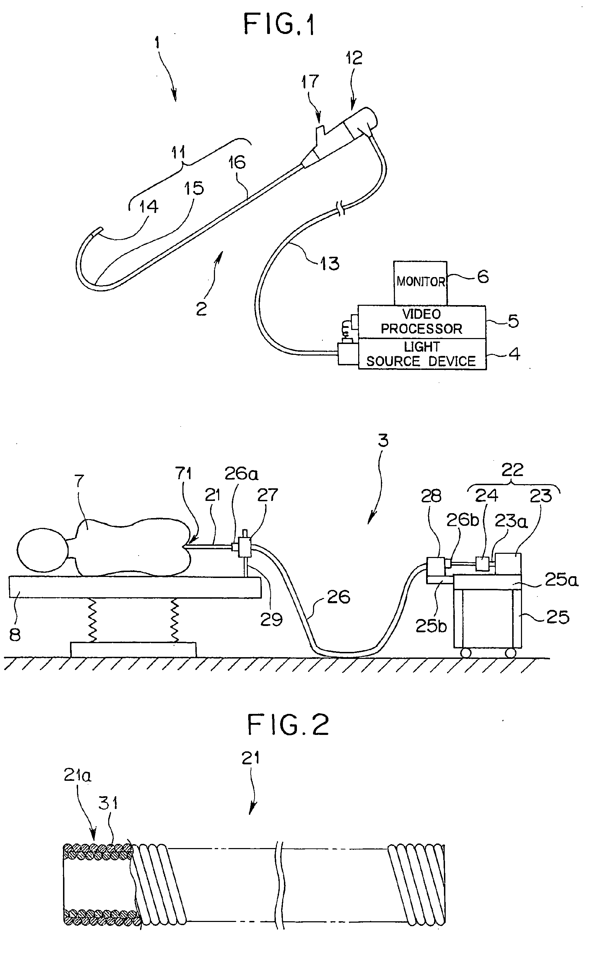

A first embodiment of an insertion device will be described with reference to Fig. 1 through Fig. 6.

As illustrated in Fig. 1, an insertion device 1 according to the present embodiment principally comprises an endoscope 2 serving as medical equipment, and an endoscope insertion assisting tool 3.

The endoscope 2 is an observation device, and comprises an insertion unit 11, an operating unit 12, and a universal cord 13. The insertion unit 11 is long, and has a length of 500 mm or longer for example. The operating unit 12 is provided at the base side of the insertion unit 11. The universal cord 13 extends from the side portion of the operating unit 12.

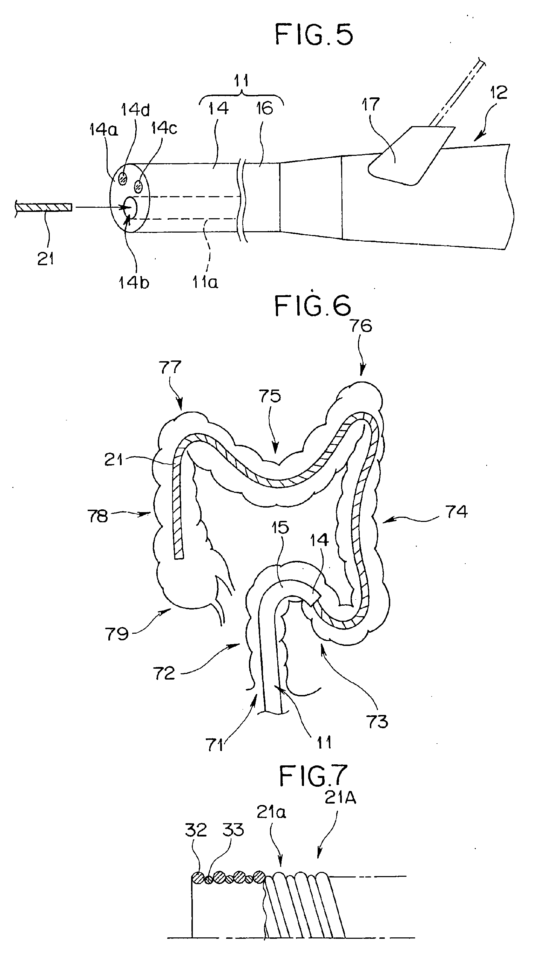

The insertion unit 11 is configured so as to serially provide a tip rigid portion 14, a bending portion 15, and a flexible tube portion 16 in order from the tip side thereof. The bending portion 15 is configured so as to bend in the vertical and horizontal directions for example. The flexible tube portion 16 has flexibility. The operating unit 12 is provided with a treatment-tool entrance 17. The treatment-tool entrance 17 is communicated with a treatment-tool insertion channel (see reference numeral 11 a in Fig. 5) serving as an insertion tube path for inserting a treatment tool provided within the insertion unit 11.

The endoscope 2 comprises a light source device 4, a video processor 5, and a monitor 6, which serve as external devices. The light source device 4 supplies illumination light to the endoscope 2. The video processor 5 includes a signal processing circuit, supplies a driving signal for driving an unshown image capturing device provided in the endoscope 2, and also generates a picture signal from the electric signal photoelectric-converted and transmitted by the image capturing device to output this to the monitor 6. An endoscope image is displayed on the screen of the monitor 6 in response to the picture signal output from the video processor 5.

The endoscope insertion assisting tool 3 is an insertion assisting member, and principally comprises a guide tube 21, and a guide-tube rotating device 22, which are insertion-unit guide members, for example. The guide-tube rotating device 22 includes a motor 23 serving as a rotating unit, and a guide-tube fixing unit 24. The motor 23 rotates the guide tube 21 in a predetermined direction of rotating on the longitudinal axis of the guide tube (hereinafter, referred to as axial rotating). The motor 23 is disposed near a bed 8 where a patient 7 lies. For example, the motor 23 is installed on the pedestal 25a of a rotating-device cart (hereinafter, abbreviated to as cart) 25. Specifically, the motor 23 is fixed on the pedestal 25a with an unshown fixing member such that the motor shaft 23a of the motor 23 becomes parallel to the upper plane of the pedestal 25a.

The motor shaft 23a of the motor 23 is configured so as to be fixed integrally with the guide-tube fixing unit 24. The guide-tube fixing unit 24 fixed to the motor shaft 23a is configured so as to be detachably attached with a base side end portion serving as one end portion of the guide tube 21.

Accordingly, upon driving the motor 23, and then rotating the motor shaft 23a, the guide tube 21 attached to the guide-tube fixing unit 24 fixed integrally to the motor shaft 23 a rotates on the shaft.

Note that reference numeral 26 denotes a protective tube for preventing the guide tube 21 from coming into contact with the floor in the operating room. The guide tube 21 is inserted into the inner hole of the protective tube 26 in a loosely fit state. Thus, the guide tube 21 is prevented from directly coming into contact with the floor or the like. End portions 26a and 26b of the protective tube 26 are detachably attached and fixed to protective-tube holding members 27 and 28 respectively. The one protective-tube holding member 27 is disposed on the bed 8 via, for example, a stand 29 of which height and position can be adjusted. The other protective-tube holding member 28 is disposed on a table 25b provided in the cart 25, facing the motor 23. A recessed member including flexibility with the longitudinal upper face side in the drawing being opened, such as a gutter shape, may be employed instead of the above protective tube 26.

The guide tube 21 illustrated in Fig. 2 is a so-called insertion unit to be inserted into a body cavity, and is, for example, a spiral tube in which ease of insertion into a body cavity is considered. The guide tube 21 is formed so as to have predetermined flexibility by winding a metal wire 31 made of stainless steel having a predetermined diameter dimension in a spiral shape to form two layers. Accordingly, the outer surface of the guide tube 21 is provided with a spiral-shaped portion 2 1 a formed with the surface of the metal wire 31.

Note that multiple (e.g., quadruple) spirals of the metal wire 31 may be wound to form the guide tube 21. Also, when winding the metal wire 31 in a spiral shape, a degree of density between metal wires is improved, and a spiral angle is changed, whereby the properties of the guide tube 21 can be set variously. Further, the outside diameter dimension of the guide tube 21 is set so as to be inserted into the treatment-tool insertion channel 11a of the endoscope 2.

Operations of the insertion device 1 thus configured will be described.

Description will be made regarding a preparatory procedure for inserting the guide tube 21 into the large intestine.

In the event of inserting the insertion unit 11 of the endoscope 2 into, for example, the appendix of the large intestine, a medical worker (abbreviated as staff) first prepares the protective tube 26, and the guide tube 21 having desired ease of insertion. Next, the staff fixes the respective end portions of the protective tube 26 to the protective-tube holding members 27 and 28 respectively. Subsequently, the staff inserts the guide tube 21 into the inner hole of the protective tube 26. Subsequently, the staff attaches one end portion of the guide tube 21 protruded from the protective tube 26 to the guide-tube fixing unit 24 fixed to the motor shaft 23a, and disposes the other end portion on the stand 29 for example. Thus, preparation for inserting the guide tube 21 into the large intestine is completed. Note that the staff prepares for the endoscope insertion assisting tool 3, and also prepares for the endoscope 2, light source device 4, video processor 5, and monitor 6.

A procedure for inserting the guide tube 21 into the large intestine will be described.

First, as illustrated in Fig. 1, a surgeon (not shown) grips the tip side portion of the guide tube 21, and inserts the tip portion of the guide tube 21 into the large intestine from the anus 71 of the patient 7 lying on the bed 8. Then, the spiral-shaped portion 2 1 a formed on the outer surface of the guide tube 21 comes into contact with the intestinal wall. At this time, the contact state between the spiral-shaped portion 21 a formed on the guide tube 21 and a fold of the intestinal wall is the relation between a male screw and a female screw.

In this contact state, the surgeon makes the transition of the motor 23 of the guide-tube rotating device 22 to a rotational driving state. Then, the guide-tube fixing unit 24 rotates, and the guide tube 21 attached to the guide-tube fixing unit 24 performs predetermined rotation. Then, as illustrated with the arrow in Fig. 3, the spiral-shaped portion 21a of the guide tube 21 is in a state rotated in the axial rotating direction such as moving from the base side to the tip side.

Thus, propulsion for advancing the guide tube 21 such as a male screw moving as to a female screw is generated at the contact portion between the rotated spiral-shaped portion 21 a of the guide tube 21 and a fold of the intestinal wall. That is to say, the spiral-shaped portion 21a is an insertion unit, and is also a propulsion generating unit. Then, the guide tube 21 proceeds to a deep portion within the large intestine, which is driven by the propulsion. At this time, the surgeon may perform a manual operation so as to press the gripped guide tube 21 forward. Note that the propulsion may be assisting of the manual operation for pressing the guide tube 21 forward.

The guide tube 21 inserted from an anus 71 advances toward a sigmoid colon portion 73 from a rectum 72 by the above propulsion, and the manual operation by the surgeon. Then, as illustrated in Fig. 3, the guide tube 21 reaches the sigmoid colon portion 73. At this time, the contact length between the spiral-shaped portion 21 a of the guide tube 21 and the intestinal wall is long. Accordingly, the stable propulsion can be obtained even in a state in which a part of the spiral-shaped portion 2 1 a is in contact with a fold of the sigmoid colon portion 73, or in a state in which the guide tube 21 is bent intricately. In addition, the guide tube 21 has sufficient flexibility, so that the guide tube 21 smoothly advances along the intestinal wall to pass through the sigmoid colon portion 73 without changing the course of the sigmoid colon portion 73 of which position readily changes.

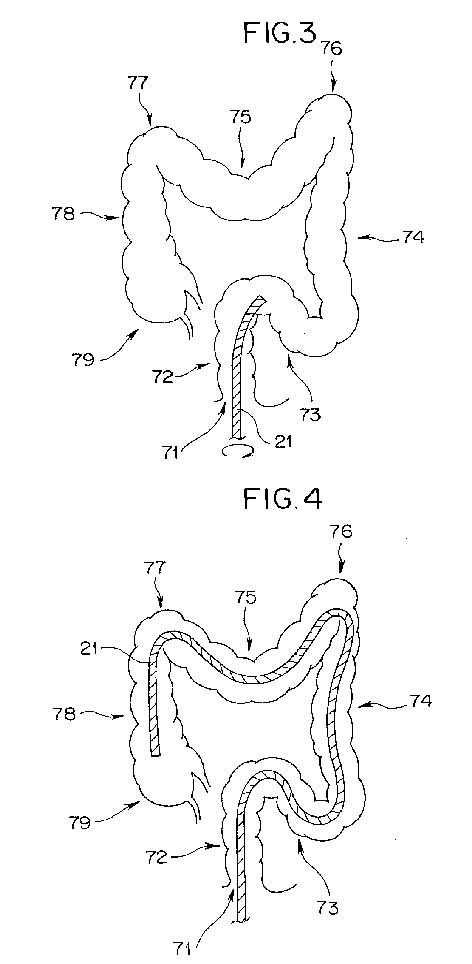

Subsequently, the guide tube 21 in a rotating state passes through a bending portion which is the boundary between the sigmoid colon portion 73 and a descending colon portion 74 where mobility is poor, passes through a splenic flexure 76 which is the boundary between the descending colon portion 74 and a transverse colon portion 75 where mobility is good, and smoothly advances along the wall of the hepatic colic flexure 77 which is the boundary between the transverse colon portion 75 and the ascending colon portion 78. Then, the guide tube 21 reaches, for example, near the appendix 79 which is the target portion, without changing the course of the large intestine, as illustrated in Fig. 4.

When the surgeon determines that the guide tube 21 has reached near the appendix 79, the staff removes the base portion of the guide tube 21 protruding from the protective tube 26 from the guide-tube fixing unit 24 under the surgeon's instruction. Then, the staff extracts the guide tube 21 from the protective tube 26.

Note that with the present embodiment, the rotational direction of the guide tube 21 which is rotated by the guide-tube rotating device may be set to only one direction (forward direction), or may be set to left or right rotation in a certain cycle or switched arbitrarily. The guide tube repeats advancing and retreating within a body cavity by combining left and right rotations of the guide-tube rotating device. Thus, even if the tip of the guide tube should get stuck with a fold or a small hollow of the intestine when advancing, engagement thereof is released when retreating. When advancing next, the guide tube can advance without getting stuck with the fold or small hollow of the intestine again by the position of the intestine shifting from the position of the guide tube subtly.

A procedure for inserting the insertion unit 11 of the endoscope 2 into the large intestine will be described.

The surgeon is inserting the base portion of the guide tube 21 extracted from the protective tube 26 toward the operating unit 12 side from a tip opening 14b communicated with the treatment-tool insertion channel 11a provided on the tip face 14a of the tip rigid portion 14, as illustrated with the arrow in Fig. 5. Subsequently, the surgeon extrudes the base portion of the guide tube 21 from the treatment-tool entrance 17 provided in the operating unit 12, such as illustrated with the dashed line in the drawing.

Upon confirming that the guide tube 21 protrudes for a predetermined amount from the treatment-tool entrance 17, the surgeon makes the transition of the endoscope 2 to an observable state to insert the insertion unit 11 into the large intestine. Subsequently, the surgeon inserts the tip rigid portion 14 making up the insertion unit 11 into the great intestine from the anus 71 in a state in which the guide tube 21 is inserted into the treatment-tool insertion channel 11 a of the insertion unit 11. Then, an observation image within the large intestine cast by the illumination light emitted from an illumination window 14c provided on the tip face of the tip rigid portion 14 is formed on the image capturing surface of the image capturing device through an observation window 14d making up an observation unit, and an endoscope image including the image of the guide tube 21 is displayed on the screen of the monitor 6.

Here, the surgeon inserts the insertion unit 11 as illustrated in Fig. 6 while confirming the extending direction of the guide tube 21 inserted into the large intestine on the screen of the monitor 6, and while performing an operation for bending the bending portion 15, an operation for twisting the insertion unit 11, or the like. At this time, the guide tube 21 inserted into the large intestine beforehand serves as a indicator indicating the insertion direction of the insertion unit 11, so that the surgeon can smoothly perform insertion work toward a deep portion of the large intestine without mistaking the insertion direction. Then, the tip rigid portion 14 of the insertion unit 11 is inserted into near the appendix 79.

Upon confirming from the endoscope image displayed on the screen of the monitor 6 that the insertion unit 11 has reached near the appendix 79 which is a target portion, the surgeon makes the transition to a retraction operation of the insertion unit 11 to perform endoscopy within the large intestine. At this time, the surgeon performs endoscopy in a state in which the guide tube 21 is inserted into the treatment-tool insertion channel 11a, or a state in which the guide tube 21 is extracted from the treatment-tool insertion channel 11a.

Note that description will be made regarding a case wherein the guide tube 21 has been returned to the anus side from the deep portion of the large intestine in a state in which the insertion unit 11 of the endoscope 2 is inserted into the large intestine. In such a case, the surgeon attaches the base portion of the guide tube 21 to the guide-tube fixing unit 24 fixed to the motor shaft of the motor 23 in a state in which the guide tube 21 is inserted into the treatment-tool insertion channel 1 1a. Then, the surgeon performs the above operation for inserting the guide tube 21 into the large intestine again to have the guide tube 21 reach the appendix 79 for example, and performs the operation for inserting the insertion unit 11 of the endoscope 2 into the large intestine again.

Thus, the surgeon inserts the guide tube into a target portion within the large intestine beforehand, and then inserts the base portion side of the guide tube provided outside of the body into the treatment-tool insertion channel provided in the endoscope, and inserts the insertion unit in a state in which the guide tube is inserted into the treatment-tool insertion channel into the large intestine. Thus, the surgeon can insert the insertion unit toward a deep portion while performing observation of the guide tube inserted and disposed within the large intestine through the observation window of the endoscope. Accordingly, the surgeon can insert the insertion unit without mistaking the insertion direction where the surgeon inserts the insertion unit. In addition, the surgeon can insert the insertion unit while performing an appropriate bending operation or twisting operation by performing observation of an insertion state of the guide tube. Thus, the surgeon can smoothly perform insertion of the insertion unit up to a deep portion of a lumen in a short period of time.

Also, with the endoscope insertion assisting tool made up of the guide tube and the guide-tube rotating device, the contact state between the spiral-shaped portion of the guide tube and the folds of the intestinal wall in a state in which the guide tube is inserted into the large intestine for example becomes a so-called relation between a male screw and a female screw by providing the spiral-shaped portion on the outer surface of the guide tube. Then, the motor of the guide-tube rotating device is rotated and driven in this contact state to rotate the guide tube in the axial rotating direction, and thus the rotating force of the guide tube is converted into propulsion, and the guide tube in a rotating state can proceed to a deep portion of the large intestine such as a male screw moving as to a female screw.

With the present embodiment, description has been made with the lumen through which the insertion unit of the endoscope is inserted being the large intestine, but the lumen through which the insertion unit is inserted is not restricted to the large intestine, and accordingly, a lumen such as the oral cavity, esophagus, stomach, small intestine, or the like may be employed.

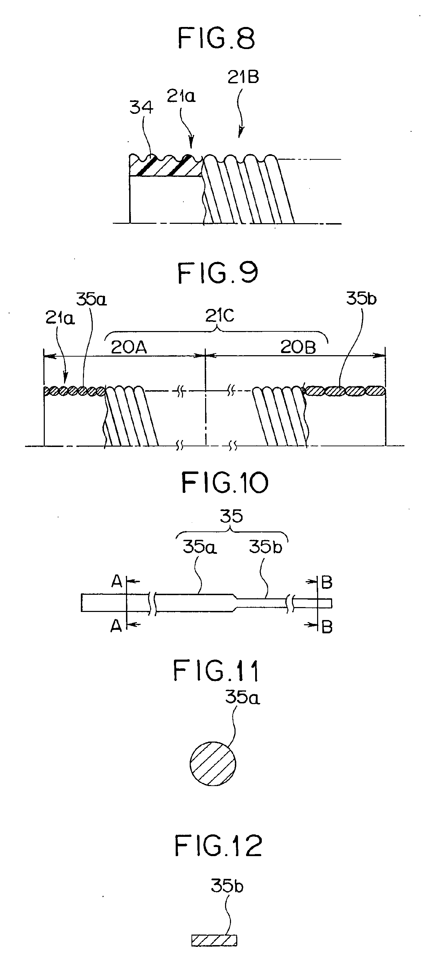

Also, with the present embodiment, the guide tube 21 is taken as a spiral tube which is configured of the metal wire 31 having a predetermined diameter dimension being wound in a spiral manner so as to form two layers, but the configuration of the guide tube 21 is not restricted to this, and accordingly, any configuration such as illustrated in the following Fig. 7 through Fig. 16 may be employed.

Description will be made regarding another configuration example of the guide tube with reference to Fig. 7 through Fig. 16.

With the guide tube 21 A illustrated in Fig. 7, for example, a single spiral is wound to form a spiral-shaped portion 21a by combining two types of metal wires 32 and 33 of which wire diameters differ. With this configuration, the wire diameters of the metal wires 32 and 33 are selected and set as appropriate.

Thus, with the guide tube 21 A, the spiral-shaped portion 2 1 a is made up of the two types of metal wires 32 and 33 of which wire diameters differ. Accordingly, propulsion to be generated when the spiral-shaped portion 21a comes into contact with a fold of the intestinal wall can be adjusted by changing the sizes of convex and recessed portions forming the spiral-shaped portion 21 a as appropriate.

The guide tube 21B illustrated in Fig. 8 is configured of a flexible resin pipe member 34 configured of the spiral-shaped portion 21a being provided by molding beforehand, or a flexible resin pipe member 34 making up the spiral-shaped portion 2 1 a by subjecting the outer face of a pipe member formed of a flexible resin member to cutting process without winding the metal wire in the spiral manner as described above.

Thus, the guide tube 21B is configured of the flexible resin pipe member 34 including the spiral-shaped portion 21a. Accordingly, inexpensive guide tubes can be obtained by molding, whereby disposal guide tubes can be realized. Also, the spiral-shaped portion 21a is formed by molding, cutting, and so forth, whereby modification of the shape, pitch, and so forth of the spiral-shaped portion 21 a can be readily performed. Accordingly, a guide tube including the spiral-shaped portion 21a appropriate for a lumen, or a guide tube including the spiral-shaped portion 21a having a shape which is desired by a surgeon can be provided.

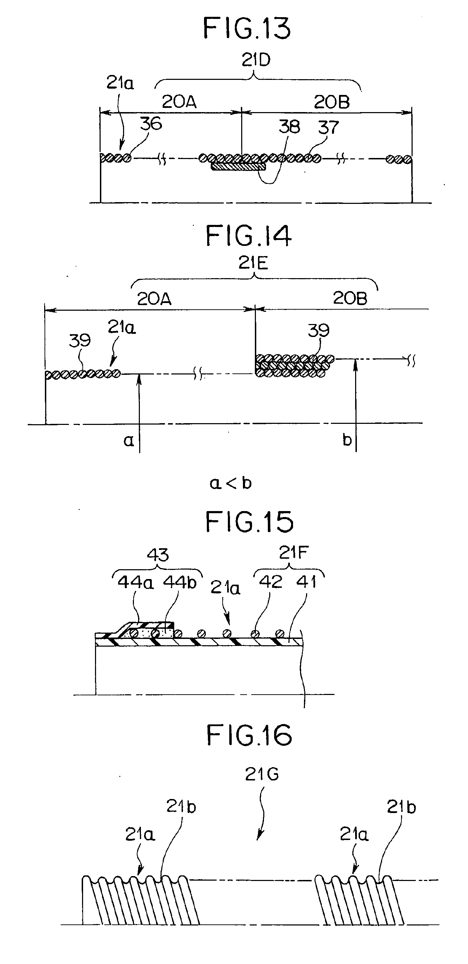

With the guide tube 21C illustrated in Fig. 9, the flexibility of a first guide tube portion 20A making up from the tip to the half-way portion to be inserted into a body cavity, and the flexibility of a second guide tube portion 20B making up the half-way portion to the base to be disposed outside of the body cavity are made to differ. Specifically, the flexibility of the first guide tube portion 20A is flexibly configured as compared with the flexibility of the second guide tube portion 20B. Accordingly, a metal wire 35 illustrated in Fig. 10 making up the guide tube 21C is made up of a tip side portion 35a of which cross-sectional shape is formed with a circle which is readily bent as illustrated in Fig. 11, and a base side portion 35b of which cross-sectional shape is formed with a square which is not flexible such as illustrated in Fig. 12.

Thus, with the guide tube 21C, an arrangement is made wherein the flexibility of the second guide tube portion 20B which is configured so as to be disposed outside a body cavity is harder than the flexibility of the first guide tube 20A to be inserted into the body cavity. Accordingly, transferability of rotating force toward the tip portion side of the guide tube 21C can be greatly improved as compared with the above guide tubes 21, 21 A, and 21B of which flexibility is evenly configured. Also, with the guide tube 21C, the first guide tube portion 20A can be smoothly inserted into a deep portion of an intricate lumen and the second guide tube portion 20B can be smoothly inserted into the treatment-tool insertion channel 11a of the endoscope 2.

Note that the arrangement wherein the flexibility of the first guide tube portion 20A making up from the tip to the half-way portion to be inserted and disposed into a body cavity, and the flexibility of the second guide tube portion 20B making up from the half-way portion to the base to be disposed outside of the body cavity are changed is not restricted to the cross-sectional shapes of the metal wire 35 illustrated in the above Fig. 9 through Fig. 12.

For example, with the guide tube 21D illustrated in Fig. 13, the flexibility of the first guide tube portion 20A, and the flexibility of the second guide tube portion 20B are changed using different two types of metal wires 36 and 37. Specifically, with the metal wire 36 making up the first guide tube portion 20A and the metal wire 37 making up the second guide tube portion 20B, flexibility is changed, for example, depending on differences of wire diameters, properties of a material, thermal processing, and so forth. Reference numeral 38 denotes a connecting fixing member 38 for connecting the different metal wires 36 and 37 integrally.

Also, with a guide tube 21E illustrated in Fig. 14, while employing the same metal wire 39, the flexibility of the first guide tube portion 20A, and the flexibility of the second guide tube portion 20B are changed by changing the number of layers of a spiral portion configured by winding the metal wire. In this case, the outside diameter dimension of the first guide tube portion 20A is formed with a diameter thinner than the outside diameter dimension of the second guide tube portion 20B.

Further, an arrangement may be made wherein with the guide tube 21B made up of a resin illustrated in Fig. 8, the flexibility of the first guide tube portion 20A, and the flexibility of the second guide tube portion 20B are changed by changing the thickness of the tube, though not shown in the drawing. In this case also, as with the above guide tube, the outside diameter dimensions are changed.

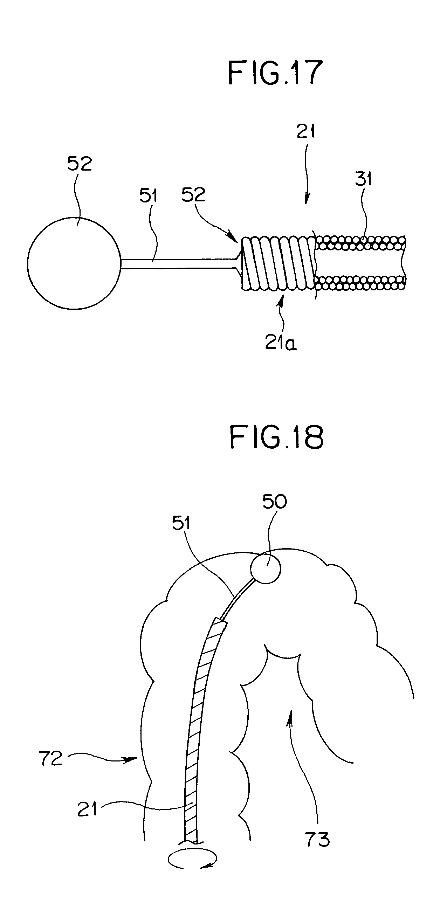

The above guide tubes 21, 21A, 21B, 21C, 21D, and 21E illustrate configurations
in which a wire is wound, and a spiral is provided on a resin member. However, as illustrated in Fig. 15, a guide tube 21 F may be configured of a rough wrap-around coil 42 making up the spiral-shaped portion 21a being disposed so as to cover a tube body 41. Reference numeral 43 is a fixing portion for fixing the end portion of the rough wrap-around coil 42 to the tube body 41 integrally. The end portion of the rough wrap-around coil 42 is integrally fixed to the tube body 41 with a fixing tape 44a and a bonding agent 44b.

With the above guide tubes 21, 21A, 21B, 21C, 21D, 21E, and 21F, the spiral-shaped portion 21 a is continuously provided across the entire length. However, as illustrated in Fig. 16, with the first guide tube portion 20A, an arrangement is made wherein multiple spiral body portions 21b including the spiral-shaped portion 21 a are partially provided at an equal interval or an arbitrary interval in light of the propulsion of the spiral-shaped portion 21 a. Thus, with a guide tube 21G, the contact portion between the spiral-shaped portion 21 a and the body wall is reduced, thereby reducing load upon the body wall.

As described above, multiple spiral-shaped portions are partially or continuously provided, whereby the contact area between the inner wall of a lumen and the spiral-shaped portion can be secured. Thus, improvement of the propulsion of the guide tube can be realized.

A second embodiment of an insertion device will be described with reference to Fig. 17 through Fig. 28.

The configuration of the insertion device according to the present embodiment is generally the same configuration as the above first embodiment, but a configuration wherein a tip guide member is provided at the tip side of an insertion-unit guide member is different from the first embodiment. Accordingly, with regard to generally the same configuration as the above first embodiment, drawings and detailed description thereof will be omitted, and description will be made below regarding only different members.

Description will be made regarding the configuration of the guide tube 21 including a tip guide member with reference to Fig. 17.

As illustrated in the drawing, the guide tube 21 is a spiral tube in which flexibility is considered, and is formed by winding a metal wire 31 in a spiral manner so as to form two layers. Accordingly, the outer surface of the guide tube 21 is provided with a spiral-shaped portion 21 a made up of the surface of the metal wire 31.

The tip portion of the guide tube 21 according to the present embodiment is provided with a guider 50 making up a tip guide member, and a wire shaft member (hereinafter, abbreviated as simply wire member) 51. The wire member 51 is provided so as to extend from the tip of the guide tube 21. Specifically, the wire member 51 is fixed to the tip portion of the guide tube 21 integrally with a fixing portion 52 made by, for example, brazing or the like. The guider 50 is a general sphere provided on the tip of the wire member 51.

The wire member 51 is fixed to the tip of the guide tube 21 with the fixing portion 52 so as to have the longitudinal center axis on the same axis as the longitudinal center axis of the guide tube 21. Accordingly, the wire member 51 is provided concentrically with the guide tube 21. The wire member 51 has a length of 10 mm to 100 mm, and is formed of a single metal wire, for example, such as stainless steel, piano wire, or the like. Also, the wire member 51 has higher flexibility than the guide tube 21. In other words, the wire member 51 is configured so as to be more flexible than the guide tube 21.

Note that the wire member 51 is not restricted to the above single metal wire, and may be a metal stranded wire. Also, the material of the wire member 51 may be a petroleum compound such as plastic, or an elastic member such as rubber, which has predetermined flexibility. Further, the wire member 51 may be a super-elastic alloy wire. Employing super-elastic alloy wire as the wire member 51 enhances restoring force which is returned to an original state even if being bent extremely. Thus, the guide tube 21 can be readily inserted in a deep portion direction within a lumen without making a U-turn.

Next, description will be made in detail regarding the guider 50 to be fixed to the tip of the wire member 51.

The guider 50 is formed of a general sphere using, for example, a metal member, and surface thereof is a smooth guide surface. The guider 50 serving as a general sphere is attached to the tip of the wire member 51 such that center point thereof is positioned on the longitudinal axis of the wire member 51. The diameter of the guider 50 is set to a predetermined diameter of a range of 2 mm to 30 mm. Also, the diameter dimension of the guider 50 is selectively set depending on either of the inside diameter of a lumen of the patient 7, or the inside diameter of the treatment-tool insertion channel 11 a of the endoscope 2.

Note that the material of the guider 50 is not restricted to a metal member, and may be a petroleum compound such as plastic having biocompatibility with surface thereof having a smooth general sphere guide face, or the like, or an elastic member such as rubber or the like. That is to say, it is desirable to have good slidability for passing over the folds of the inner wall of a lumen such as the large intestine or the like, i.e., to be a material of which frictional coefficient is small. Also, as for the guider 50, a lightweight material is preferable, and as for weight saving, the inside of the guider 50 may be hollow for example. Further, it is desirable to configure the guider 50 with a transparent resin or the like.

Description will be made regarding operations of the guide tube 21 including the guider 50 thus configured.

A preparatory procedure for inserting the guide tube 21 into the large intestine are the same as that in the above first embodiment, so description thereof will be omitted here.

Next, description will be made regarding an operation for inserting the guide tube 21 into the large intestine.

First, as illustrated in the above Fig. 1, a surgeon (not shown) grips the tip side portion of the guide tube 21, and inserts the tip portion of the guide tube 21 into the large intestine from the anus 71 of the patient 7 lying on the bed 8. Then, the spiral-shaped portion 21 a formed on the outer surface of the guide tube 21 comes into contact with the intestinal wall. At this time, the contact state between the spiral-shaped portion 21a formed on the guide tube 21 and a fold of the intestinal wall is the relation between a male screw and a female screw.

In this contact state, the surgeon rotates and drives the motor 23 of the guide-tube rotating device 22. Then, the guide tube 21 is rotated in the axial rotating direction. At this time, the guider 50 of the guide tube 21 is propagated with rotation movement from the guide tube 21 and the wire member 51, and is rotated in the axial rotating direction. Propulsion for advancing the guide tube 21, such as a male screw moving as to a female screw, is generated at the contact portion between the spiral-shaped portion 21a of the guide tube 21 which is rotated and the fold of the intestinal wall. Subsequently, the guide tube 21 proceeds to the rectum 72, and the sigmoid colon portion 73 using the propulsion.

As illustrated in Fig. 18, the guider 50 of the guide tube 21 reaches the sigmoid colon portion 73. At this time, the guide face of the guider 50 of the guide tube 21 is in contact with a fold of the sigmoid colon portion 73. While rotating in accordance with rotation of the guide tube 21, the guider 50 which is in contact with the intestinal wall of the sigmoid colon portion 73 having folds smoothly passes over the fold in contact with a part of the generally-spherical surface of the guide face. Upon the guide tube 21 further advancing within the large intestine, the guider 50 leads the guide tube 21 to the advancing direction thereof by being pressed along the bending portion of the sigmoid colon portion by the wire member 51.

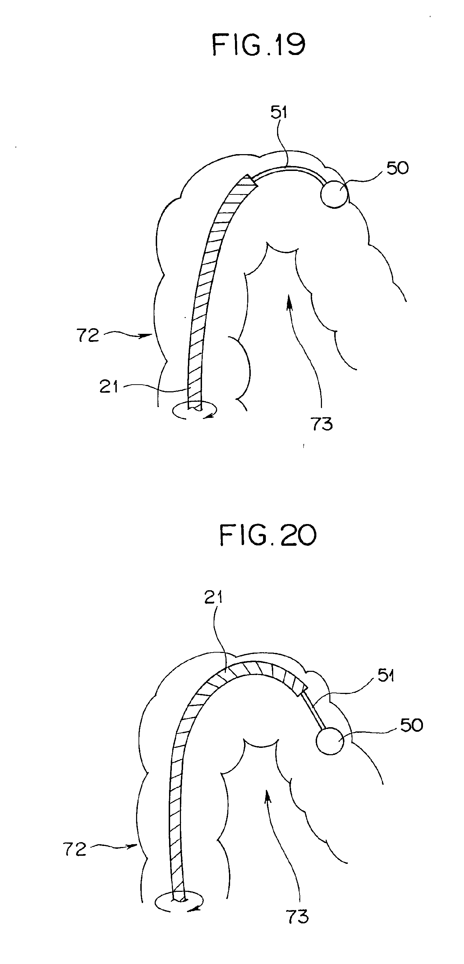

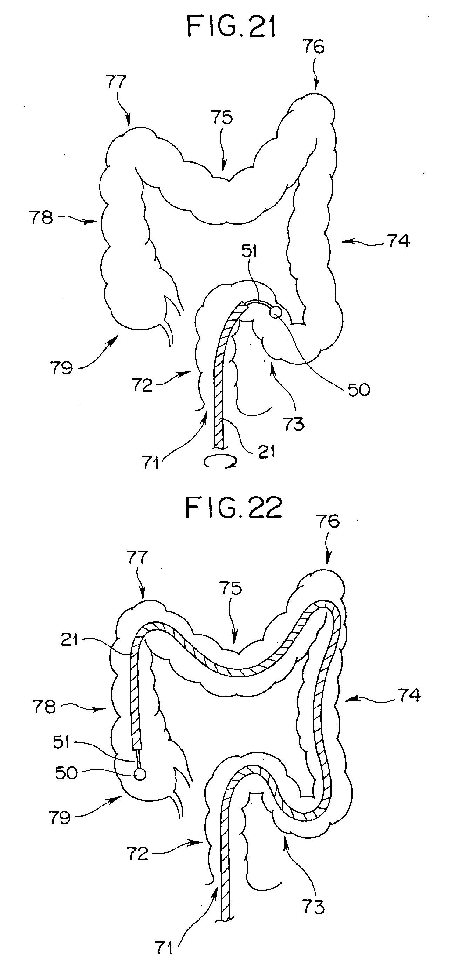

In accordance therewith, the wire member 51 is bent along the bending portion of the sigmoid colon portion as illustrated in Fig. 19, following which the tip portion of the guide tube 21 connected with the wire member 51 is similarly bent as illustrated in Fig. 20 and Fig. 21, and is led along the side wall of the sigmoid colon portion 73. Also, even in a state in which the guider 50 is in contact with the intestinal wall of the sigmoid colon portion 73, the relation between the spiral-shaped portion 21 a of the guide tube 21 and the folds of the intestinal wall is the relation between a male screw and a female screw. Accordingly, the guide tube 21 in a rotating state smoothly advances along the intestinal wall without changing the position of the very flexible sigmoid colon portion, of which position is readily changed.

Subsequently, the guide tube 21 in a rotating state passes over the folds of the intestinal wall such that the guider 50 passes through the sigmoid colon portion 73 by being dragged in the bending direction of the wire member 51. Thus, as illustrated in Fig. 22, the guide tube 21 smoothly advances along the intestinal walls of a bending portion serving as the boundary between the sigmoid colon portion 73 and the descending colon portion 74 where mobility is poor, the splenic flexure 76, which is a bending portion, serving as the boundary between the descending colon portion 74 and the transverse colon portion 75 where mobility is good, the hepatic colic flexure 77, which is a bending portion, serving as the boundary between the transverse colon portion 75 and the ascending colon portion 78, and reaches near the appendix 79 for example without changing the course of the large intestine.

With this advancing process, the contact length between the spiral-shaped portion 21a of the guide tube 21 and the intestinal wall is long, so that stable propulsion can be obtained even in a state in which the guider 50 is in contact with the intestinal wall, and even in a state in which the guide tube 21 is intricately bent. In addition, the guide tube 21 has sufficient flexibility, so that the guide tube 21 smoothly advances along the intestinal wall, for example, without changing the position of the sigmoid colon portion 73 of which position is readily changed during advancing the inside of the large intestine.

Upon the guider 50 of the tip of the guide tube 21 reaching near the appendix 79, the surgeon removes the base portion of the guide tube 21 protruding from the protective tube 26 from the guide-tube fixing unit 24. Subsequently, the surgeon extracts the guide tube 21 from the base portion side from the protective tube 26.

Procedures to be performed next, i.e., a procedure for inserting the insertion unit 11 of the endoscope 2 into the large intestine, and an endoscopy procedure within the large intestine, are completely the same as those in the above first embodiment, and accordingly, description thereof will be omitted.

Thus, with the insertion device according to the present embodiment, when inserting the guide tube into a lumen, e.g., the large intestine or the like, the guide tube can readily pass over the folds of the intestinal wall by a part of the guide face which is a general sphere of the guider provided at the tip side of the guide tube coming into contact with a fold of the intestinal wall.

Also, the wire member of the guide tube is bent in the advancing direction of the guider, whereby the guide tube can be readily inserted toward a deep portion direction along the curve of each bending portion such as the large intestine and the like.

Accordingly, the surgeon advances the insertion unit of the endoscope along the guide tube inserted into a lumen beforehand, whereby the surgeon can readily perform insertion up to a deep portion. According to the above arrangements, the surgeon can insert the insertion unit of the endoscope into a target portion smoothly and also in a short period of time without causing the patient pain.

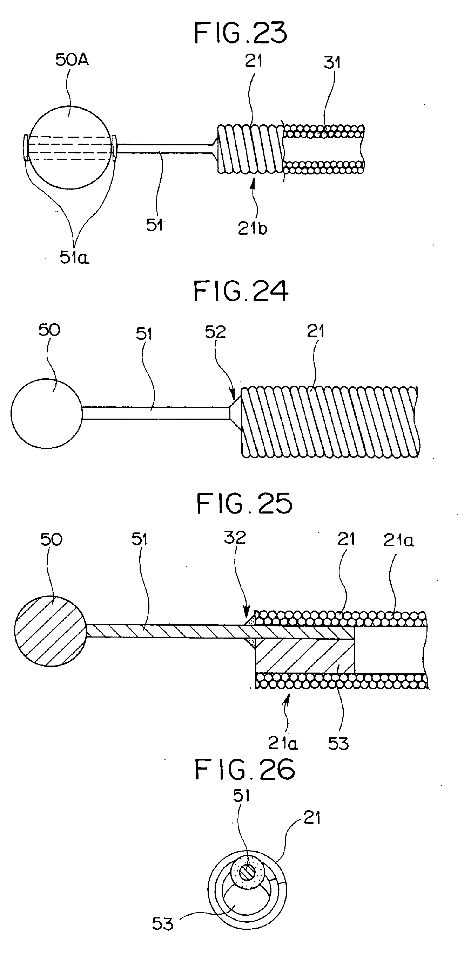

The configuration of the guider is not restricted to the above embodiment, and may be a configuration such as illustrated in Fig. 23, for example. The guider 50A according to the present embodiment includes a through hole in which the wire member 51 is inserted and disposed centered on the guider, as illustrated in Fig. 23. The tip of the wire member 51 and a predetermined position on the half way are provided with a pair of stoppers 51a for sandwiching the guider 50 so as to move rotationally. The outer shape of these stoppers 51 a is set so as to be greater than the opening of the through hole provided in the guider 50. Accordingly, the guider 50 is disposed so as to move rotationally around the longitudinal axis of the wire member 51 .

With this configuration of the guider 50A, upon the guide face serving as a surface coming into contact with the intestinal wall, the wire member 51 is rotated to no useful purpose. Also, upon the guide face reaching a bending portion such as the large intestine or the like, the guider 50 performs appropriate rotational movement. As a result, the guider 50 can readily pass over the folds of the intestinal wall without providing excessive rotational load upon the intestinal wall by the guide face. Accordingly, the guide tube 21 can more smoothly reach up to a deep portion such as the large intestine or the like.

Also, the positional relation between the wire member 51 and the guide tube is not restricted to a configuration wherein the wire member 51 and the guide tube 21 are provided concentrically as described with the above embodiment, and may be a configuration such as illustrated with Fig. 24 through Fig. 26.

With the present embodiment, as illustrated in Fig. 24, the longituidinal axis of the wire member 51 provided on the tip of the guide tube 21, and the longitudinal axis of the guide tube 21 have a different axis. That is to say, the wire member 51 is provided with eccentricity as to the center axis of the guide tube 21. In other words, the longitudinal axis of the wire member 51 is an eccentric axis shifted from the rotational axis of the guide tube 21.

As illustrated in Fig. 25 and Fig. 26, the base portion of the wire member 51 is inserted into the tip portion of the guide tube 21 so as to be sandwitched with the tube path inner face of the guide tube 21 and the surface of an eccentric member 53, and is fixed integrally by a fixing portion 52. The tip of the wire member 51 is fixed with the guider 50. The longitudinal axis of the wire member 51 is disposed so as to pass through the center of the guider 50. That is to say, the tip of the guide tube 21 is provided with the wire member 51 having the eccentric axis, and the tip of the wire member 51 is provided with the guider 50.

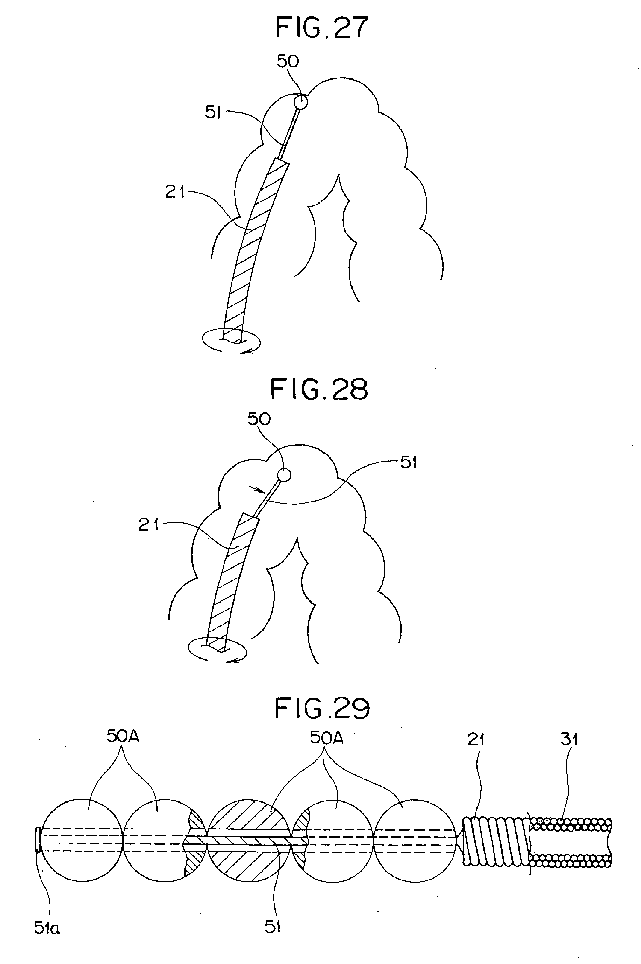

Description will be made regarding an operation of the guide tube 21 including the wire member 51 eccentric as to the center axis of the guide tube 21 with reference to Fig. 27 and Fig. 28.

The guide tube 21 is rotated in the axial rotating direction, and advances within the large intestine as if a male screw moves as to a female screw. Subsequently, as illustrated in Fig. 27, upon the guide tube 21 reaching a bending portion of the large intestine, a part of the guide face of the guider 50 comes into contact with the intestinal wall. In this state, the guide tube 21 is rotating, so as illustrated in Fig. 28, the position in the axial rotating direction of the guider 50 is shifted in accordance with the eccentric axis of the wire member 51. Accordingly, the guider 50 moves within the intestinal wall while changing the position in the axial rotating direction along with rotation of the guide tube 21, so that the guider 50 can readily smoothly pass over the folds of the intestinal wall. Accordingly, the guide tube 21 can readily reach up to a deep portion such as the large intestine and the like.

A third embodiment of an insertion device will be described with reference to Fig. 29 through Fig. 35.

The configuration of an insertion device according to the present embodiment is generally the same configuration as the above second embodiment, but the configuration of the tip guide member provided at the tip side of the insertion-unit guide member is slightly different from the second embodiment. Accordingly, with regard to the same configuration as the above first embodiment, drawings and detailed description thereof will be omitted, and description will be made below regarding only different members.

As illustrated in Fig. 29, the tip of the guide tube 21 according to the present embodiment is attached with the wire member 51. With this wire member 51, multiple, here, five guiders 50A are provided. The five guiders 50A are provided with a through hole passing through center thereof. The through holes are inserted with the wire member 51. The diameter dimension of the through holes is designed so as to be greater than the diameter of the wire member 51. Also, the tip of the wire member 51 is provided with a stopper 51 a having an outer shape which is greater than the openings of the through holes of the guiders 50A. The stopper 51a is provided for preventing the five guiders 50A from falling off the wire member 51.

Accordingly, of the five guiders 50A through which the wire member 51 is inserted, the stopper 51 a abuts on the surface at the tip opening portion side of the guider 50A at the leading edge, and the tip face side of the guide tube 21 abuts on the surface at the base opening portion side of the guider 50A at the trailing edge. Accordingly, the five guiders 50A are prevented from falling off the wire member 51, and can move rotationally as to the wire member 51, or can stop.

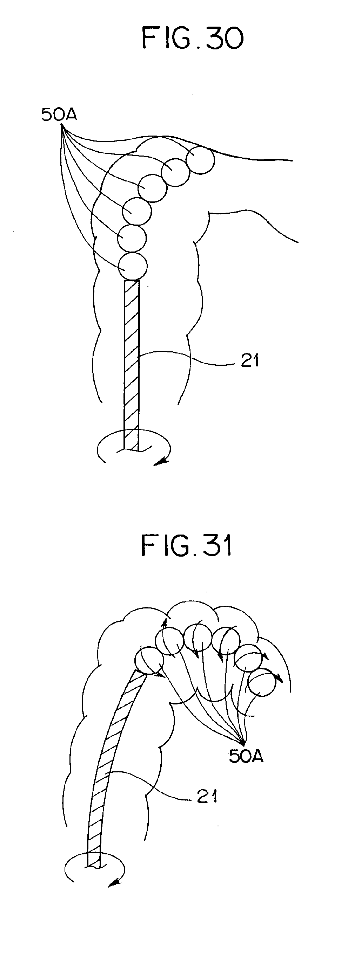

Description will be made regarding an operation of the guide tube 21 in which the multiple guiders 50A are provided with reference to Fig. 30 and Fig. 31.

The guide tube 21 is rotated in the axial rotating direction, and advances within the large intestine as a male screw moves as to a female screw. Subsequently, as illustrated in Fig. 30, a part of the guide face of the guider 50A positioned at the tip side of the guide tube 21 touches, and comes into contact with the intestinal wall such as the large intestine or the like, and particularly, the intestinal wall of a bending portion such as the sigmoid colon portion 73 (see Fig. 21). At this time, the guider 50A at the leading edge can move rotationally as to the wire member 51, so that the guider 50A at the leading edge is not rotated even if the guide tube 21 is rotated. Accordingly, the guider 50A at the leading edge does not provide wasteful load upon the intestinal wall of the large intestine and the like with which the guider 50A at the leading edge comes into contact.

Also, as illustrated in Fig. 31, the five guiders 50A which pass through the intestinal wall of a bending portion such as the large intestine or the like can move rotationally, so that influence of the wire member 51 which rotates along with the guide tube 21 is small. Accordingly, the five guiders 50A can rotate in the most appropriate direction of the axial rotating direction, or the opposite direction of the axial rotating direction as illustrated with arrows depending on a state of a fold of the intestinal wall through which the five guiders 50A pass. That is to say, the guide face of each guider 50A smoothly passes over the folds of the intestinal wall while rotating in either of the axial rotating direction, or the opposite direction of the axial rotating direction.

Subsequently, upon the guide tube 21 further advancing within the large intestine, the guiders 50A are smoothly led along a bending portion of the sigmoid colon portion 73. In accordance therewith, the wire member 51 is bent along the bending portion of the sigmoid colon portion 73, the tip portion of the guide tube 21 connected thereto is similarly bent, and is led by being dragged. That is to say, with the guide tube 21 in a rotating state, the guider 50A at the leading edge passes through each bending portion of the large intestine by being dragged in the bending direction of the wire member 51 while passing over the folds of the intestinal wall, and can reach up to a deep portion such as the large intestine or the like.

As a result of the above arrangements, in addition to the advantages of the second embodiment, upon the five guiders 50A of the guide tube 21 coming into contact with the intestinal wall, the five guiders 50A are not rotated, and particularly, with each bending portion of a lumen, the five guiders 50A can freely rotate without being influenced by rotation of the wire member 51, and accordingly, the five guiders 50A can readily pass over the folds of the intestinal wall without creating a wasteful load upon the intestinal wall.

Accordingly, the surgeon can cause the guide tube and the insertion unit 11 of the endoscope 2 to reach up to a deep portion such as the large intestine or the like smoothly. Thus, the surgeon can insert the insertion unit of the endoscope into a target portion smoothly and in a short period of time without causing a patient pain.

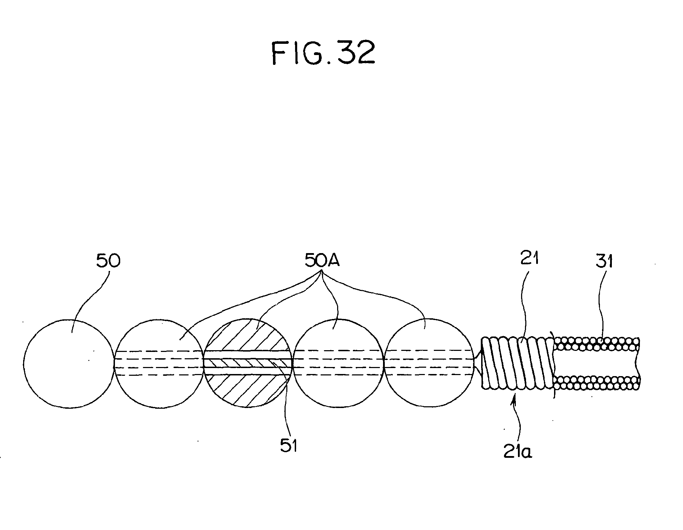

Note that as illustrated in Fig. 32, an arrangement may be made wherein when providing multiple guiders, five in this case, to the wire member 51, the guider 50 positioned at the leading edge is fixed to the wire member 51, the four guiders 50A are provided with a through hole through which the wire member 51 passes so as to pass through center thereof, and the guiders 50A other than the guider 50 at the leading edge can move rotationally around the longitudinal axis of the wire member 51.

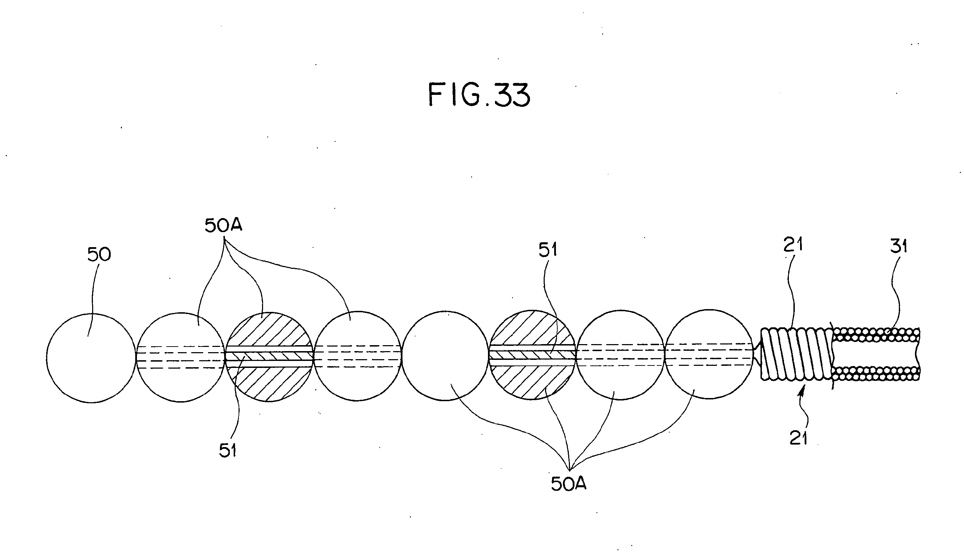

Also, as illustrated in Fig. 33, an arrangement may be made wherein for example, eight guiders are provided in the wire member 51, the two guiders 50 at the leading edge and at the intermediate position are fixed to the wire member 51, the remaining six guiders 50A are provided with a through hole through which the wire member 51 passes so as to pass through center thereof, and the six guiders 50A can move rotationally around the longitudinal axis of the wire member 51.

As for a placement example of guiders, as illustrated in Fig. 33, for example, three guiders of the six guiders 50A which can move rotationally are provided between the guiders 50 at the leading edge and at the intermediate position, the three guiders 50A other than those are provided at the base side on the wire member 51, i.e., on the wire member 51 from the guider 50 at the intermediate position to the guide tube 21. Consequently, the rotational moving force of the wire member 51 is propagated to the guiders 50 fixed to the wire member 51 which are positioned at the leading edge and at the intermediate position, thereby further improving the propulsion of the guide tube 21.

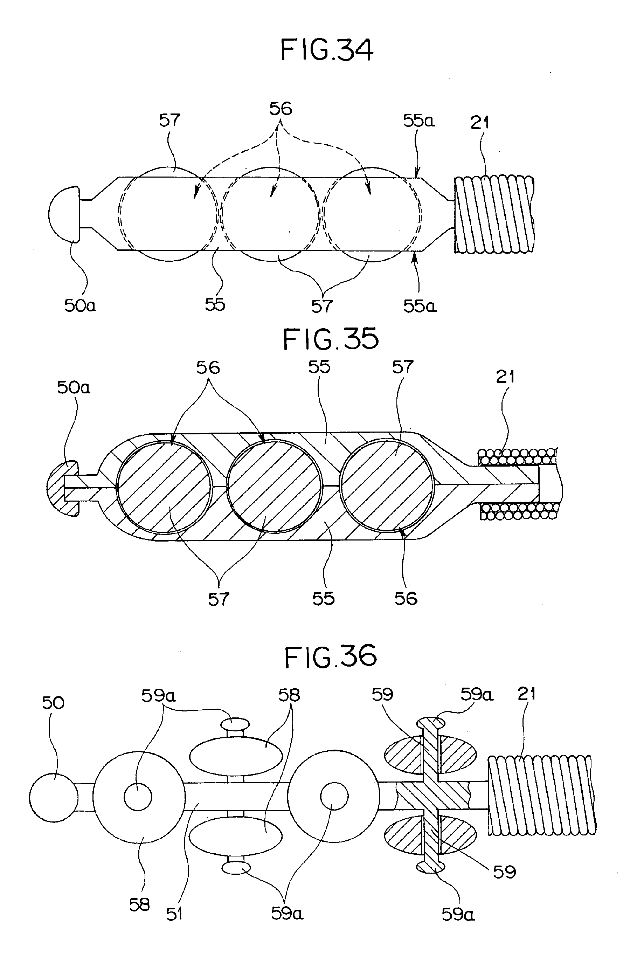

Further, the second embodiment and the third embodiment illustrate the tip guide member having a configuration wherein the wire member 51 is provided at the tip of the guide tube 21, and at least one guider is provided as to the wire member 51, but the configuration of the tip guide member is not restricted to these, and may be a configuration such as illustrated in Fig. 34 and Fig. 35.

As illustrated in Fig. 34 and Fig. 35, with the present embodiment, an arrangement is made wherein the tip side of the guide tube 21 is provided with a holding member 55 having a guide face configured of a smooth outer surface which sandwiches and holds three spheres for example. This holding member 55 is configured of two members to be divided, and the inside of each of component members includes three hole portions 56 at an equal interval. These three hole portions 56 are each provided with a sphere 57 of which surface includes a guide face. The tip of the two component members to be divided is provided with a cap 50a. The holding member 55 is configured by integrating the two divided component members with this cap 50a.

The holding member 55 has a general cylindrical shape, and is subjected to chamfering 55a in two directions of outer circumferential faces thereof facing each other. Accordingly, a part of the guide faces of the spheres 57 are exposed from the chamfers 55a included in the holding member 55. Further, the hole diameters of the three hole portions 56 are set to be slightly greater than the diameter of the spheres 57. Consequently, the spheres 57 disposed within the holding member 55 can perform various rotations. Thus, as described above, the spheres 57 can be moved rotationally, whereby the guide tube 21 can be readily inserted.

Also, as illustrated in Fig. 36, the tip of the guide tube 21 may be provided with multiple, here, four pairs of rotators 58 so as to become generally orthogonal to the longitudinal direction axis of the wire member 51. These pairs of rotators 58 have, for example, a general donut shape, and surface thereof includes a smooth guide face for passing over the folds of the intestinal wall of the large intestine or the like. A pivot 59 is passed through each of the through holes provided in the rotators 58. Both ends of these pivots 59 are provided with a retaining portion 59a which is greater than the through holes of the rotators 58. Accordingly, these rotators 58 are disposed so as to move rotationally as to the pivots 59. Also, these rotators 58 are disposed so as to have alternately the axis of the orthogonal direction from the tip side of the wire member 51. That is to say, the rotational moving axis of the adjacent rotators 58 is alternately in the general orthogonal direction.

Thus, when advancing the guide tube 21 in a deep portion direction of the large intestine, the rotators 58 are moved rotationally, whereby the fold of the intestinal wall which is in contact with the guide face of the rotator 58 can be readily passed over.

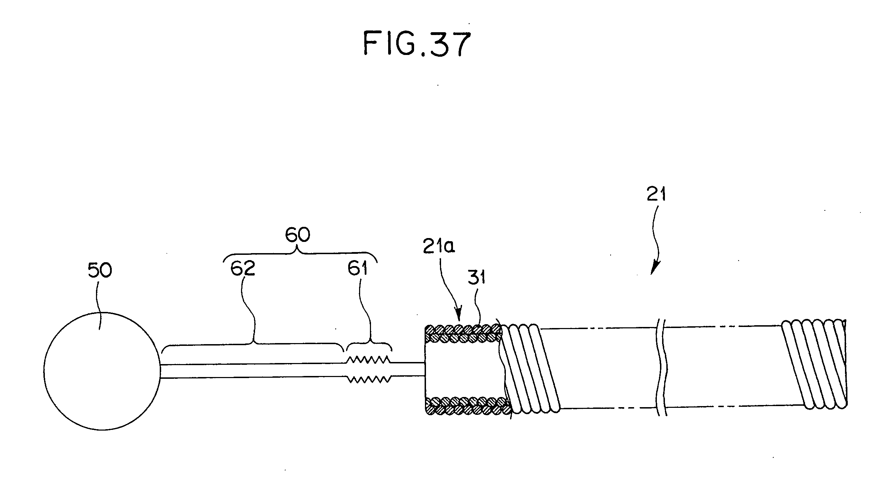

Description will be made regarding a fourth embodiment of the present invention with reference to Fig. 37 through Fig. 41.

The configuration of the insertion device according to the present embodiment is generally the same configuration as the above second embodiment, but a configuration wherein a tip guide member is provided at the tip side of an insertion-unit guide member is slightly different from the second embodiment. Accordingly, with regard to the configuration generally the same as the above second embodiment, drawings and detailed description thereof will be omitted, and description will be made below regarding only different members.

The guide tube 21 illustrated in Fig. 37 is a spiral tube in which ease of insertion into a body cavity is considered. The guide tube 21 is a tube which is formed by winding the stainless steel wire 31 having a predetermined diameter dimension in a spiral manner so as to have two layers and predetermined flexibility, for example.

The tip portion of the guide tube 21 is provided with a tip guide member made up of a guider 50 and a wire shaft member (hereinafter, abbreviated as simply wire member) 60. The wire shaft member 60 comprises a first flexible portion 61, and a second flexible portion 62. The guider 50 is a general sphere of which outer surface is smoothly formed, which makes up the leading edge of the guide member. The first flexible portion 61 is a linear member having flexibility, which is provided at the base portion side serving as a portion close to the guide tube 21. The second flexible portion 62 is a linear member having flexibility, which continuously connects the guider 50 and the first flexible portion 61.

With the present embodiment, the guide tube 21, the first flexible portion 61, and the second flexible portion 62 all have flexibility, and the first flexible portion 61 is set so as to have more flexibility than the second flexible portion 62.

That is to say, upon comparing the flexibility of both the flexible portions 61 and 62, the relation of
flexibility of the second flexible portion 62 > flexibility of the first flexible portion 61 holds.

Also, the second flexible portion 62 is set so as to have more flexibility than the guide tube 21, or so as to have generally the same flexibility as the guide tube 21.

That is to say, upon comparing the flexibility of the guide tube 21 and the flexibility of the second flexible portion 62, the relation of
flexibility of the guide tube 21 ≥ flexibility of the second flexible portion 62 holds.

Accordingly, with between the guide tube 21, the first flexible portion 61, and the second flexible portion 62, the relation of
flexibility of the guide tube 21 ≥ flexibility of the second flexible portion 62 > flexibility of the first flexible portion 61 is set.

Also, the length relation between the first flexible portion 61 and the second flexible portion 62 is set such that
length of the second flexible portion 62 ≥ length of the first flexible portion 61 holds.

Note that the diameter of the guider 50 is set to be greater than the diameter of the guide tube 21.

Description will be made regarding an operation of the guide tube in which the tip guide member to which the flexibility of the flexible portions 61 and 62 are set, as described above, is provided.

Here, the preparatory procedure for inserting the guide tube 21 into the large intestine is the same as that in the above first embodiment, so description thereof will be omitted.

Next, an operation for inserting the guide tube 21 into the large intestine will be described.

First, as illustrated in Fig. 1, a surgeon (not shown) grips the tip side portion of the guide tube 21 A, and inserts the guider 50 and the wire member 60 making up the tip guide member of the tip portion of the guide tube 21 into the large intestine from the anus of the patient 7 lying on the bed 8, and subsequently inserts the guide tube 21. Then, the spiral-shaped portion 21 a formed on the outer surface of the guide tube 21 comes into contact with the intestinal wall. At this time, the contact state between the spiral-shaped portion 21 a formed on the guide tube 21 and a fold of the intestinal wall is the relation between a male screw and a female screw.

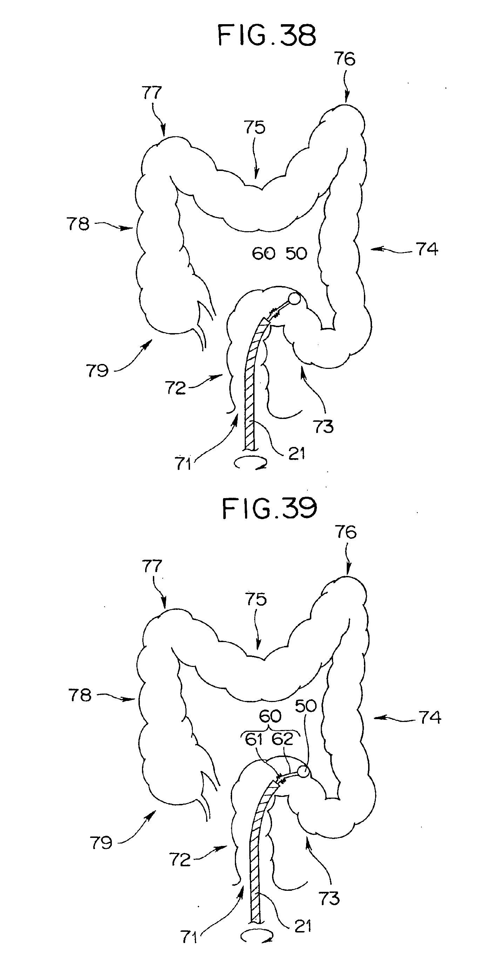

In this contact state, the surgeon makes the transition of the motor 23 of the guide-tube rotating device 22 to a rotational driving state. Then, the guide-tube fixing unit 24 rotates, and the base portion of the guide tube 21 attached to the guide-tube fixing unit 24 performs predetermined rotation. This rotation is propagated to the tip side from the base portion, and as illustrated with the arrow in Fig. 38, the spiral-shaped portion 21a of the guide tube 21 is in a state rotated in the axial rotating direction such as moving from the base side to the tip side.

Thus, propulsion for advancing the guide tube 21 such as a male screw moving as to a female screw is generated at the contact portion between the spiral-shaped portion 21a of the rotating guide tube 21 and the folds of the intestinal wall. Then, the guide tube 21 proceeds to a deep portion within the large intestine, which is driven by the propulsion. At this time, the surgeon may perform a manual operation such as pressing the gripped guide tube 21 forward.

The guide tube 21 inserted from the anus 71 proceeds to the sigmoid colon portion 73 from the rectum 72 by the above propulsion, and the manual operation by the surgeon. Then, as illustrated in Fig. 38, the guide tube 21 reaches the sigmoid colon portion 73. At this time, the contact length between the spiral-shaped portion 21 a formed on the outer surface of the guide tube 21 and the intestinal wall is long, so that the rotating guide tube 21 smoothly advances through the sigmoid colon portion 73 which is bent, and also where mobility is good. Thus, the guider 50 of the guide tube 21 is in a state of abutting on the wall face of the sigmoid colon portion 73.

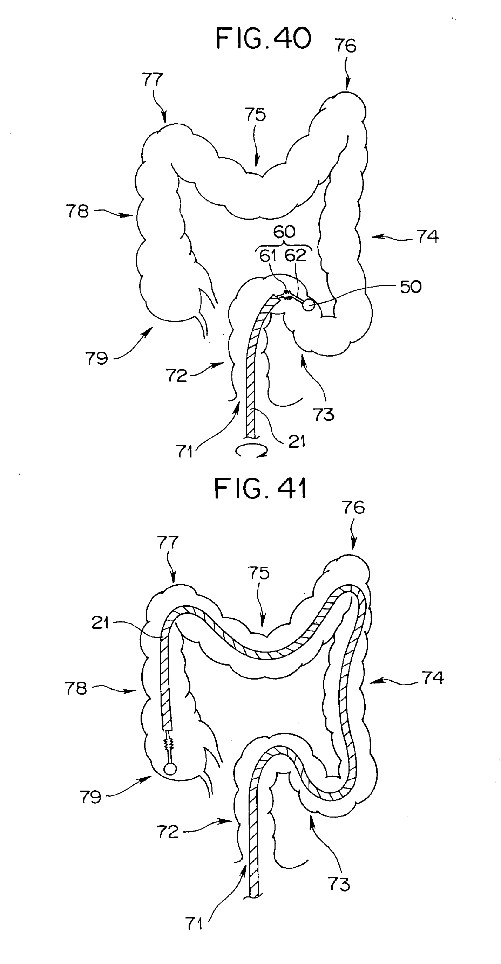

In this state, upon predetermined propulsion being further provided upon the guide tube 21, the state is changed into the state illustrated in Fig. 39. In this state, the first flexible portion 61 is bent, and simultaneously, the guider 50 advances in the insertion direction along the wall face by further pressing the guide tube 21 forward from the state in which the guider 50 is in contact with the wall face of a bending portion of the sigmoid colon portion 73 (see the above Fig. 38). At this time, the second flexible portion 62 is formed harder than the first flexible portion 61, so that the second flexible portion 62 serves in advancing the guider 50 along the wall face. Further, upon the guide tube 21 being pressed forward, the guider 50 smoothly advances along the wall face as illustrated in Fig. 40.

Subsequently, the guider 50 passes through the sigmoid colon portion 73, and the guide tube 21 also smoothly advances in the same direction in accordance with this. In this advancing state, even if the guide tube 21 is pressed toward the wall face excessively, the first flexible portion 61 is bent depending on the wall face. Thus, the guider 50 can smoothly advance, of which advancement is not disturbed due to the guider 50 intruding into irregularities to be formed on the wall face of the intestinal tract, or the like.

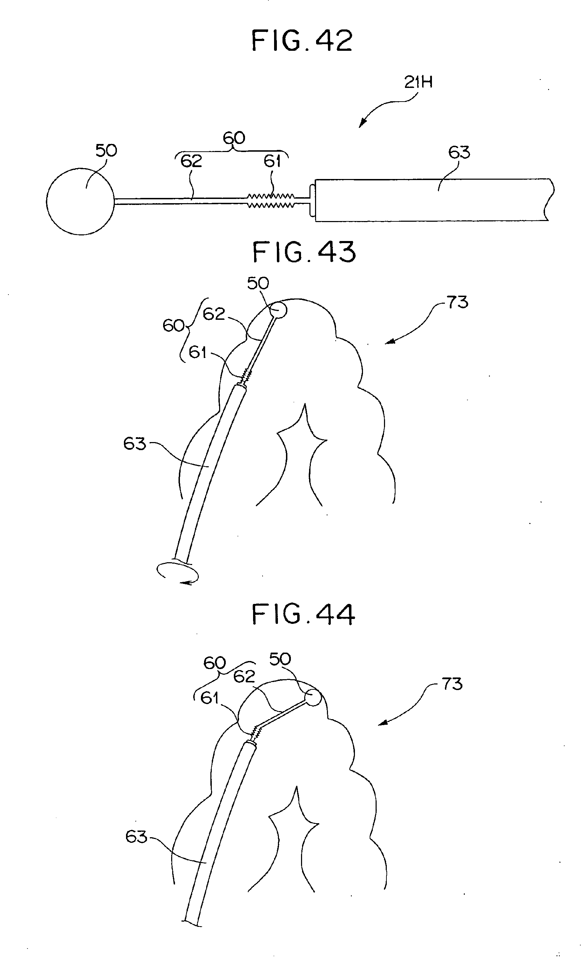

Then, the guide tube 21 in a rotating state passes through the sigmoid colon portion 73, and subsequently advances smoothly along the walls of a bending portion which is the boundary between the sigmoid colon portion 73 and the descending colon portion 74 where mobility is poor, the splenic flexure 76 which is the boundary between the descending colon portion 74 and the transverse colon portion 75 where mobility is good, and the hepatic colic flexure 77 which is the boundary between the transverse colon portion 75 and the ascending colon portion 78, and reaches near the appendix 79 which is a target portion without changing the course of the large intestine, as illustrated in Fig. 41.

When the surgeon determines that the tip guide member of the guide tube 21 has reached near the appendix 79, the staff removes the guide tube 21 from the protective tube 26 under the surgeon's instruction. Then, the staff makes the transition to work for inserting the insertion unit 11 of the endoscope 2 into the large intestine.

Procedures to be performed next, i.e., a procedure for inserting the insertion unit 11 of the endoscope 2 into the large intestine, and an endoscopy procedure within the large intestine, are completely the same as those in the above first embodiment, and accordingly, description thereof will be omitted.

Thus, when providing the tip guide member at the tip portion of the guide tube, like the present embodiment, the wire member making up the tip guide member is set so as to have different flexibility depending on portion thereof. Thus, in a state in which the guide tube is inserted into the large intestine for example, in the event that the guide tube is further advanced by propulsion following the guider abutting on the wall face of the intestinal tract, the first flexible portion is bent, and in accordance with this, the guider can be smoothly advanced along the wall face.

Note that as illustrated in Fig. 42, an arrangement may be made wherein a guide tube 21H is configured so as to have the outer surface which is not made up of the spiral-shaped portion 21 a, i.e., at least a part of the outer surface of a portion which can be in contact with a subject in light of only ease of insertion into a body cavity is made up of a slender flexible tube body 63 which is subjected to hydrophilic polymer coating for improving lubricity, for example.

Description will be made regarding an operation of the guide tube in which the tube body 63 is provided with a tip guide member of which flexibility is thus set.

Here, a preparatory procedure for inserting the guide tube 21H into the large intestine is the same as that in the above fourth embodiment, so description thereof will be omitted.

Next, an operation for inserting the guide tube 21H into the large intestine will be described.

First, as illustrated in the above Fig. 1, a surgeon (not shown) grips the tip side portion of the guide tube 21A, and inserts the guider 50 making up the tip guide member of the tip portion of the guide tube 21 and the wire member 60 into the large intestine from the anus of the patient 7 lying on the bed 8, and subsequently inserts the guide tube 21. Then, a part of the outer surface of the tube body 63 making up the guide tube 21H comes into contact with the intestinal wall. At this time, the moisture of the human body and hydrophilic polymer are bound, a watery film is formed on the surface of the tube body 63, thereby improving lubricity as to the large intestine.

In this contact state, the surgeon places the motor 23 of the guide-tube rotating device 22 in a rotational driving state. Then, the guide-tube fixing unit 24 rotates, and the base portion of the guide tube 21H attached to the guide-tube fixing unit 24 performs predetermined rotation. This rotation is propagated to the tip side from the base portion, and as illustrated with the arrow in Fig. 43, the tube body 63 of the guide tube 21H is in a state rotated in the axial rotating direction. At this time, the surgeon performs a manual operation so as to press the gripped guide tube 21H forward.

Thus, the outer surface of the rotated tube body 63 comes into contact with the folds of the intestinal wall across the entire circumference. Then, the lubricity of the tube body 63 improves, and the guide tube 21H smoothly proceeds to a deep portion within the large intestine by the manual operation.

Then, as illustrated in Fig. 43, the guide tube 21H reaches the sigmoid colon portion 73. At this time, the guider 50 of the guide tube 21H is in a state of contact with the wall face of a bending portion of the sigmoid colon portion 73. In this state, upon the guide tube 21H being further pressed forward, the state is changed into the state illustrated in Fig. 44. At this time, the first flexible portion 61 is bent, and simultaneously, the guider 50 advances in the insertion direction along the wall face by the surgeon further pressing the guide tube 21H forward from the state in which the guider 50 is in contact with the wall face of the bending portion of the sigmoid colon portion 73 illustrated in Fig. 43. At this time, the second flexible portion 62 is formed harder than the first flexible portion 61, so that the second flexible portion 62 serves in advancing the guider 50 along the wall face.

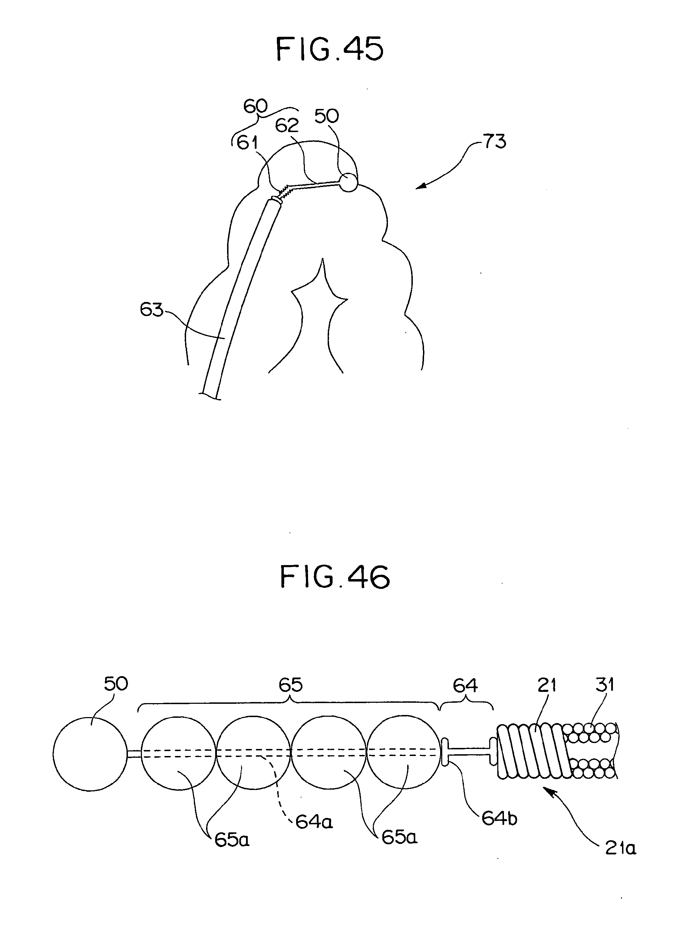

From this state, the surgeon performs a manual operation for further pressing the guide tube 21H forward. Then, as illustrated in Fig. 45, the guider 50 smoothly advances along the wall face, and passes through the sigmoid colon portion 73. Then, the guide tube 21H also smoothly advances in the same direction in accordance with movement of the guider 50. In this state, even if the surgeon excessively presses the guide tube 21H forward, the first flexible portion 61 is bent and deformed depending on the wall face. Thus, the guider 50 can smoothly advance, of which advancement is not disturbed such as the guider 50 getting stuck in a recessed portion of folds formed on the wall face of the intestinal tract, or catching in a protrusion, or the like.

Thus, the guide tube is configured of the tube body in which ease of insertion within a lumen is considered, and the rotating force of the guide tube is converted into propulsion by rotating and driving the motor of the guide-tube rotating device to rotate the guide tube in the axial rotating direction in this contact state, whereby the guide tube in a rotating state can be proceeded to a deep portion of the large intestine such as a male screw moving as to a female screw.

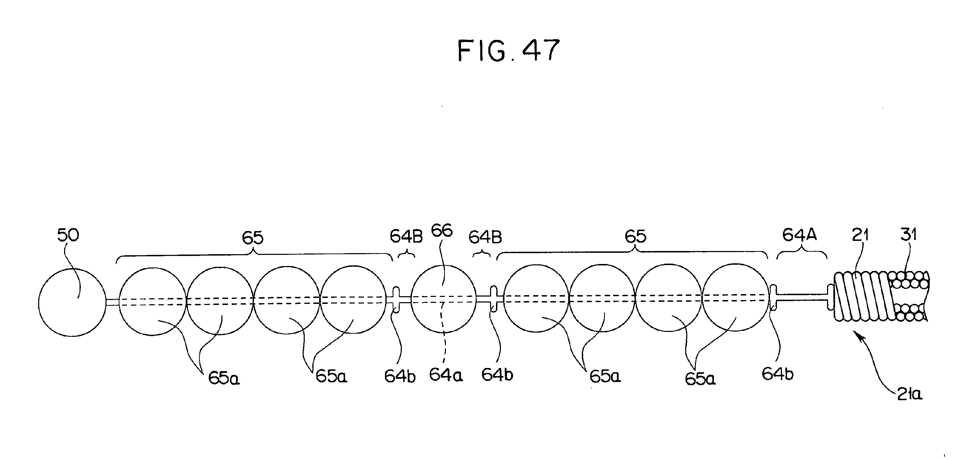

Note that the configuration of the tip guide member in which flexibility of the insertion-unit guide tube is set is not restricted to the above fourth embodiment, and may be configured such as illustrated in Fig. 46 or Fig. 47. Note that drawings and detailed description thereof will be omitted regarding the same configurations as the fourth embodiment, and description will be made below regarding only different members.

As illustrated in Fig. 46, with the present embodiment, the tip guide member fixedly provided at the tip of the guide tube 21 comprises a guider 50 formed in the same way as that in the fourth embodiment, a first flexible portion 64 made up of a linear member, and a second flexible portion 65 which is formed with multiple general spheres being consecutively provided, which are integrally consecutively provided.

With the present embodiment, the guider 50 and the first flexible portion 64 are consecutively provided with the same linear member 64a extending from the above first flexible portion 64. The linear member 64a passes through multiple (four in the present embodiment) general spheres 65a making up the second flexible portion 65, and holds these in a skewered state. A stopper 64b is provided at the interface portion between the first flexible portion 64 and the second flexible portion 65.

The flexibility of the guide tube 21, the flexibility of the first flexible portion 64, and the flexibility of the second flexible portion 65 according to the present embodiment are set completely in the same way as the relation between the respective members according to the above fourth embodiment.

That is to say, upon comparing the flexibility of the first flexible portion 64, and the flexibility of the second flexible portion 65, the relation of flexibility of the second flexible portion 65 > flexibility of the first flexible portion 64 holds.

Also, upon comparing the flexibility of the guide tube 21 and the flexibility of the second flexible portion 65, the relation of
flexibility of the guide tube 21 ≥ flexibility of the second flexible portion 65 holds.

Accordingly, with between the flexibility of the guide tube 21, the flexibility of the first flexible portion 64, and the flexibility of the second flexible portion 65, flexibility is set with the relation of flexibility of the guide tube 21 ≥ flexibility of the second flexible portion 65 > flexibility of the first flexible portion 64.

Also, the length relation between the first flexible portion 64 and the second flexible portion 65 is set such that the relation of
length of the second flexible portion 65 ≥ length of the first flexible portion 64 holds.

Further, the diameter of the guider 50 is set to be greater than the diameter of the guide tube 21.

Operations of the guide tube 21 including the flexible portions 65 and 64 thus configured are completely the same as those in the above fourth embodiment. Also, the advantages obtained from these are the same. Further, the second flexible portion 65 of which flexibility is different from the first flexible portion 64 can be configured by disposing only the general spheres 65a on the linear member 64a formed of the same material as the first flexible portion 64. Accordingly, this can contribute to improvement of productivity, and reduction in manufacturing costs.

On the other hand, with the present embodiment such as illustrated in Fig. 47, the tip guide member fixedly provided at the tip of the guide tube 21 comprises the above guider 50 provided at the leading edge portion, a joint portion 66, first flexible portions 64A and 64B made up of a linear member, and a pair of second flexible portions 65 which are formed with multiple general spheres being consecutively provided, which are integrally consecutively provided. The joint portion 66 is a mobile coupling portion made up of a general sphere similar to the guider 50.

With the present embodiment, the guider 50 and the first flexible portion 64 are consecutively provided with the same linear member 64a extending from the above first flexible portion 64. This linear member 64a passes through multiple (4 x 2 = 8 in the present embodiment) general spheres 65a making up a pair of the second flexible portions 65, and holds these in a skewered state.

A stopper 64b is provided at the interface portion between the first flexible portion 64 and the second flexible portion 65 at the base side. The stopper 64b is also provided at the interface portion between the second flexible portions 65 at the base side and the joint portion 66. The stopper 64b is also provided at the interface portion between the joint portion 66 and the second flexible portions 65 at the tip side. The above members are arrayed in the sequence of the guide tube 50, second flexible portion 65, first flexible portion 64B, joint portion 66, first flexible portion 64B, second flexible portion 65, and first flexible portion 64A from the leading edge side. That is to say, with the tip guide member according to the present embodiment, a pair of the second flexible portions 65 are disposed sandwiching the joint portion 66, thereby providing two portions which can be bent.

Here, the flexibility of the guide tube 21, the flexibility of the first flexible portions 64A and 64B, and the flexibility of the second flexible portions 65 according to the present embodiment are set completely in the same way as the relation between the respective members according to the above first embodiment. That is to say, upon comparing the flexibility of the first flexible portions 64A and 64B, and the flexibility of the second flexible portions 65, the relation of
flexibility of the second flexible portions 65 > flexibility of the first flexible portions 64A and 64B
holds.

Also, upon comparing the flexibility of the guide tube 21 and the flexibility of the second flexible portions 65, the relation of
flexibility of the guide tube 21 ≥ flexibility of the second flexible portions 65 holds.

Accordingly, with between the flexibility of the guide tube 21, the flexibility of the first flexible portions 64A and 64B, and the flexibility of the second flexible portion 65, flexibility is set with the relation of
flexibility of the guide tube 21 ≥ flexibility of the second flexible portions 65 > flexibility of the first flexible portions 64A and 64B.

Also, the length relation between the first flexible portions 64A and 64B and the second flexible portions 65 is set such that the relation of
length of the second flexible portions 65 ≥ length of the first flexible portion 64A > length of the first flexible portion 64B
holds.

Further, the diameter of the guider 50 is set to be greater than the diameter of the guide tube 21.

Operations of the guide tube 21 including the flexible portions 65, 64B, 64B, 65, and 64A thus configured are as follows.

The preparatory procedure for inserting the guide tube 21 into the large intestine is the same as the above embodiments, so description thereof will be omitted.

Next, a procedure for inserting the guide tube 21 into the large intestine will be described.

First, a surgeon (not shown) grips the tip side portion of the guide tube 21, and inserts the guide tube 21 including the guider 50 and the flexible portions 65, 64B, 64B, 65, and 64A into the large intestine from the anus of the patient 7 lying on the bed 8. Then, the guider 50 of the guide tube 21 comes into contact with the intestinal wall.

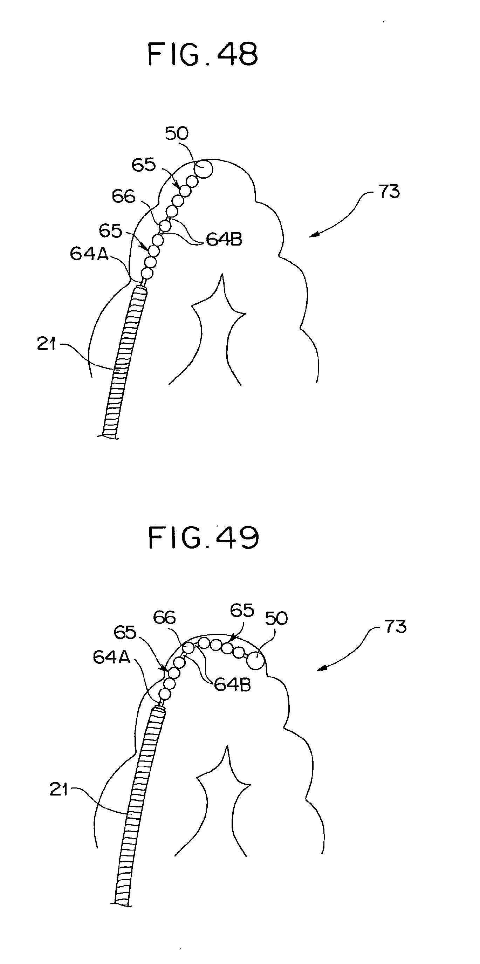

In this contact state, the surgeon rotates and drives the motor of the guide-tube rotating device, grips the guide tube 21, and advances this toward the inside of the body cavity. Thus, the guide tube 21 advances toward a deep portion within the large intestine. Subsequently, the guide tube 21 inserted from the anus advances toward the sigmoid colon portion 73 from the rectum by propulsion and operations at the location of the surgeon, and reaches the sigmoid colon portion 73 as illustrated in Fig. 48. In the state illustrated in Fig. 48, the guider 50 protruding at the tip of the guide tube 21 is in contact with the wall face of a bending portion of the sigmoid colon portion 73. From this state, upon the surgeon further advancing the guide tube 21 forward, the state is changed into the state illustrated in Fig. 49.

At this time, the guide tube 21 is advanced forward from the state in which the guider 50 is in contact with the wall face of the bending portion of the sigmoid colon portion 73, and thus, the first flexible portion 64B is bent centered on the joint portion 66. Also, simultaneously therewith, the guider 50 advances in the insertion direction along the wall face, and the joint portion 66 is in a state of contact with the wall face. Here, a pair of the second flexible portions 65 are formed so as to be harder than the first flexible portions 64A and 64B, so that this serves in advancing the guider 50 along the wall face.

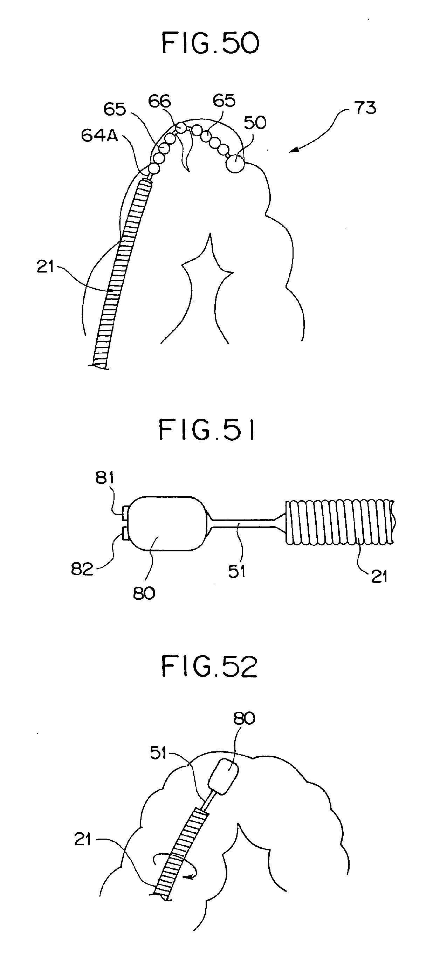

Upon the surgeon further advancing the guide tube 21 forward, the guider 50 smoothly advances along the wall face such as illustrated in Fig. 50. Subsequently, the guider 50 passes through the sigmoid colon portion 73, and in accordance with this, the guide tube 21 also smoothly advances in the same direction. In this state, even if the surgeon excessively presses the guide tube 21 forward, the first flexible portions 64A and 64B are bent depending on the wall face, and thus, the guider 50 can smoothly advance to reach near the appendix which is a target portion for example, of which advancement is not disturbed such as the guider 50 getting stuck in a recessed portion to be formed on the wall face of the intestinal tract, or catching in a protrusion, or the like.

When the surgeon determines that the tip guide member of the guide tube 21 has reached near the appendix, the staff removes the guide tube 21 from the protective tube 26 under the surgeon's instruction. Then, the staff makes the transition to work for inserting the insertion unit 11 of the endoscope 2 into the large intestine.

The procedure to be performed next, i.e., a procedure for inserting the insertion unit 11 of the endoscope 2 into the large intestine is completely the same as that in the above embodiment, and accordingly, description thereof will be omitted.

As described above, with the present embodiment, an arrangement is made wherein a pair of the second flexible portions 65 are disposed sandwiching the joint portion 66, whereby this can be effectively utilized for more intricate bending portions.

Thus, in the event of employing an insertion assisting member of a type for inserting the insertion unit of an endoscope such as a later-described so-called overtube (also referred to as sliding tube) into the inside thereof, the outside diameter of a portion to be inserted into a subject is the outside diameter of the overtube. On the contrary, in the event of employing an arrangement wherein the above insertion assisting member (guide tube 21) is inserted into the treatment-tool insertion channel of an endoscope, the outside diameter of a portion to be inserted into a subject is the outside diameter of the insertion unit of the endoscope. Accordingly, a portion to be inserted into a subject is prevented from being thicker than the overtube, and in the event that a subject is a patient, the patient can be prevented from being forced with much burden.

Also, the tip portion of the insertion assisting member is configured so as to advance and retreat as to the observational visual field region of an observation unit, thereby enabling the insertion assisting member to be disposed outside the observational visual field region of the observation unit when observing a subject, or when performing deeds, for example, such as subjecting the target portion of a subject to medical treatment, or the like. Consequently, the insertion assisting member can be prevented from becoming hindrance of deeds such as observation, treatment, or the like as much as possible.

Description will be made regarding a fifth embodiment of the present invention with reference to Fig. 51 through Fig. 61.

With the present embodiment, the guider 30 is a capsule-type endoscope 80, and this configuration differs from the above embodiments. Accordingly, with regard to the same configurations as the above embodiments, drawings and detailed description thereof will be omitted, and only different members will be described below.

The capsule-type endoscope 80 illustrated in Fig. 51 is configured so as to have surface thereof serving as a smooth guide face. The tip side of the capsule-type endoscope 80 includes an observation window 81 making up an observation unit for capturing images, and an illumination window 82 for casting illumination light.

The base side of the wire member 51 to which the capsule-type endoscope 80 is fixed is fixed to the guide tube 21. Accordingly, the capsule-type endoscope 80 rotates in accordance with rotating of the guide tube 21. Accordingly, during rotating of the guide tube 21, there is risk of a picture to be displayed on the screen of the unshown monitor rotating in accordance with rotating thereof. In order to prevent this problem, with the present embodiment, an arrangement is made wherein rotational correction processing of a picture to be displayed on the monitor is performed in sync with the rotational cycle of the guide-tube rotating device 22 using the unshown video processor so as to display an ordinary endoscope image on the screen of the monitor.

Note that this capsule-type endoscope 80 may be a disposable type or a reusable type. Also, the guide face which is the outer surface of the capsule-type endoscope 80 may be subjected to processing for improving lubricity.

Operations of the guide tube 21 of the insertion device 1 according to the present embodiment will be described.

Note that the preparation for inserting the guide tube 21 into the large intestine, and the like is the same as those in the above embodiments, so description thereof will be omitted.

First, a surgeon inserts the capsule-type endoscope 80 side of the guide tube 21 into the large intestine from the anus 71 in a state in which the patient 7 is lying on the bed 8. Then, the outer circumferential face of the guide tube 21 is in a state of contact with the intestinal wall. Here, the surgeon rotates and drives the motor 23 of the guide-tube rotating device 22. Then, the guide tube 21 is rotated in the axial rotating direction, and advances within the large intestine such as a male screw moving as to a female screw.

Then, as illustrated in Fig. 52, a part of the guide face of the capsule-type endoscope 80 positioned at the tip side of the guide tube 21 touches and comes into contact with the intestinal wall of the large intestine or the like, e.g., the intestinal wall of a bending portion such as the sigmoid colon portion or the like. At this time, rotation of the guide tube 21 is propagated to the wire member 51, so that the capsule-type endoscope 80 is in a rotating state in accordance with rotation of the guide tube 21. At this time, a picture captured through the observation window 81 of the capsule-type endoscope 80 is subjected to rotational correction processing in sync with the rotational cycle of the guide-tube rotating device 22 by the video processor, thereby being displayed on the screen of the monitor as an ordinary endoscope image. Thus, the surgeon can precisely determine a state of the capsule-type endoscope 80 as to the intestinal wall from above the screen, whereby the guide tube 21 can be smoothly advanced toward a deep portion of the large intestine by the surgeon performing the most appropriate manual operation in addition to propulsion.

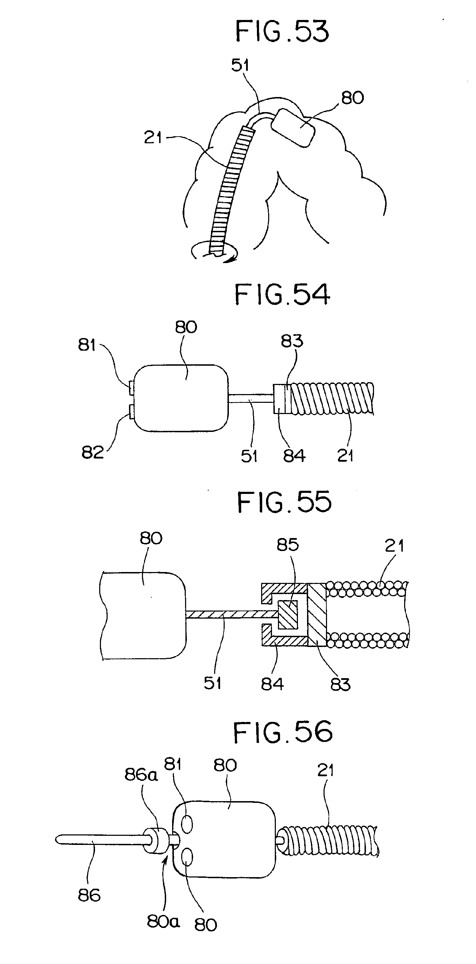

Also, as illustrated in Fig. 53, the capsule-type endoscope 80 which passes through the intestinal wall of a bending portion such as the large intestine or the like is propagated with rotation of the wire member 51 which rotates along with the guide tube 21 in accordance with a state of the fold of the intestinal wall through which the capsule-type endoscope 80 passes, thereby rotating in the axial rotating direction. Accordingly, the guide face of the capsule-type endoscope 80 smoothly passes over the folds of the intestinal wall while rotating in the axial rotating direction.

Upon the guide tube 21 further advancing within the large intestine, the capsule-type endoscope 80 is smoothly led along a bending portion of the sigmoid colon portion, therewith the wire member 51 is bent along the bending portion of the sigmoid colon portion. Then, the tip portion of the guide tube 21 connected with the wire member 51 is similarly bent, and is led by being dragged.

Subsequently, with the guide tube 21 in a rotating state, the capsule-type endoscope 80 passes through each bending portion of the large intestine by being dragged in the bending direction of the wire member 51 while passing over the folds of the intestinal wall. Then, upon confirming on the screen that the capsule-type endoscope 80 positioned at the tip of the guide tube 21 has reached near the appendix, the surgeon performs a retraction operation of the guide tube 21 for example to perform endoscopy within the large intestine. Subsequently, the surgeon performs endoscopy within the large intestine while retracting the guide tube 21.

Thus, according to the present embodiment, the surgeon can perform endoscopic observation without inserting the insertion unit 11 of the endoscope 2 into a lumen of the patient 7 again following inserting the guide tube 21 into a predetermined portion such as the above embodiment.

Accordingly, the surgeon can readily bring the guide tube 21 including the capsule-type endoscope 80 up to a deep portion such as the large intestine or the like, and also can perform endoscopic observation smoothly in a short period of time without causing the patient 7 pain. Also, the capsule-type endoscope 80 is disposed at more forward side than the tip face of the guide tube 21, thereby preventing a field of view from being hindered by the guide tube 21 in a sure manner.

Note that with the present embodiment, in Fig. 51, the observation window 81 and the illumination window 82 are in a state of being protruded from the tip side guide face of the capsule-type endoscope 80 for the sake of facilitating description, but the observation window 81 and the illumination window 82 are in a face-matching state as to the tip side guide face.

Also, as illustrated in Fig. 54, an arrangement may be made wherein the tip portion of the guide tube 21 is provided with a supporting member 83 and a falling-off-prevention member 84. The falling-off-prevention member 84 supports the wire member 51 fixed to the tip of the capsule-type endoscope 80 so as to be moved rotationally, and also serves as prevention from falling off.

Specifically, as illustrated in Fig. 55, the falling-off-prevention member 84 has a cylindrical shape, and a thorough hole where the wire member 51 is inserted and disposed is formed at the bottom center of internal space thereof. A falling-off-prevention portion 85 is arranged to be disposed at the base portion of the wire member 51. The outer shape of the falling-off-prevention portion 85 is set so as to be greater than the diameter dimension of the through hole. Accordingly, the wire member 51 is prevented from falling off the guide tube 21 in a state in which the falling-off-prevention portion 85 is disposed inside the falling-off-prevention member 84.

Note that the falling-off prevention member 84 is configured so as to be fixed to the supporting member 83 of the guide tube 21 following the wire member 51 being inserted and disposed. According to this configuration, the capsule-type endoscope 80 fixed to the tip of the wire member 51 can be moved rotationally as to the guide tube 21.

Consequently, even if the outside diameter of the capsule-type endoscope 80 becomes greater, excessive rotational load, i.e., excessive friction due to the guide face of the capsule-type endoscope 80, can be prevented from being applied to the intestinal wall in a sure manner. Also, the picture captured through the observation window 81 of the capsule-type endoscope 80 is not rotated along with rotation of the guide tube 21, whereby image processing for displaying an ordinary picture on the monitor can be readily performed as compared with the case of performing image processing in sync with the rotational cycle of the guide-tube rotating device 22 using the unshown video processor.

Note that the configuration of the guide tube 21 including the capsule-type endoscope 80 is not restricted to the above embodiments, e.g., an arrangement may be made wherein as illustrated in Fig. 56, with the capsule-type endoscope 80, a longitudinal-direction through hole 80a passing through center thereof is provided, and a flexible portion 86 fixed so as to protrude from the tip of the guide tube 21 is passed through the longitudinal-direction through hole 80a. The flexible portion 86 is a rod-shaped member having high flexibility. A stopper 86a for preventing the capsule-type endoscope 80 disposed in this flexible portion 86 from falling off is provided in the flexible portion 86. In this drawing, the tip guide member comprises the capsule-type endoscope 80 and the flexible portion 86.

According to this configuration, even if the shape of the capsule-type endoscope 80 is great, the flexible portion 86 guides this so as to be in complying with the intestinal wall in the advancing direction. Accordingly, resistance to be generated between the tip side guide face of the capsule-type endoscope 80 and the intestinal wall can be reduced. Also, the tip portion of the flexible portion 86 protruding more leading side than the capsule-type endoscope 80 is observed through the observation window 81 of the capsule-type endoscope 80, thereby displaying the image of the flexible portion 86 on the screen of the monitor. Accordingly, the surgeon can confirm the advancing direction of the guide tube 21 by confirming the flexible portion 86 protruding from the capsule-type endoscope 80, whereby more improvement of ease of insertion can be realized.

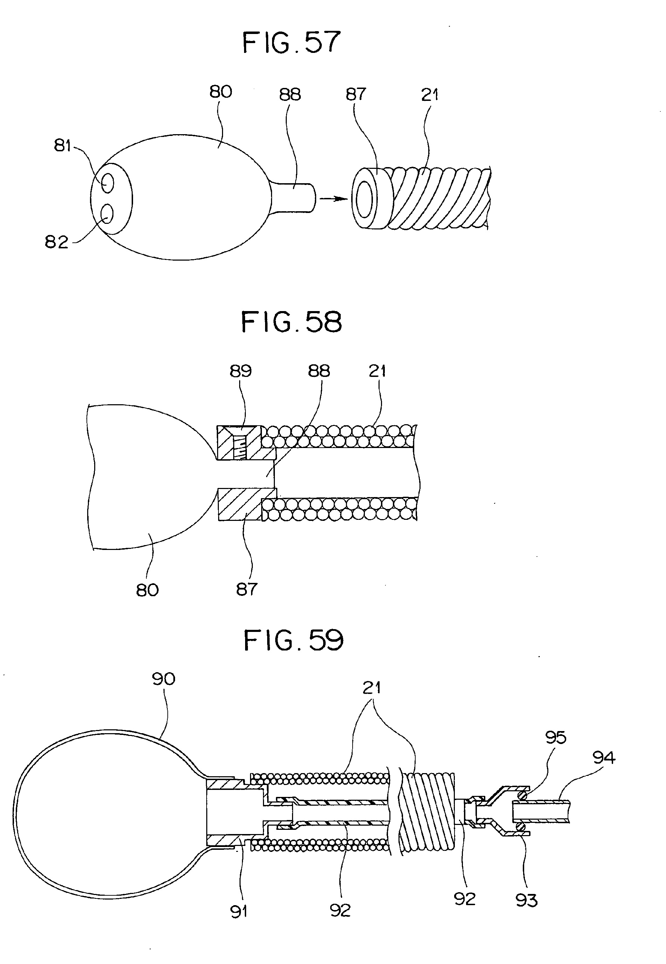

Also, as illustrated in Fig. 57, an arrangement may be made wherein a ring-shaped fixing member 87 is provided at the tip portion of the guide tube 21, and the capsule-type endoscope 80 is attached to the fixing member 87. In this configuration also, the capsule-type endoscope 80 is disposed more forward side than the tip face of the guide tube 21, whereby a field of view can be prevented from being hindered by the guide tube 21 in a sure manner.

With the capsule-type endoscope 80, surface thereof has a smooth guide face, and tip side thereof includes an observation window 81 for capturing images, and an illumination window 82 for casting illumination light. The base side of the capsule-type endoscope 80 is provided with a fixing protrusion 88 to be inserted into the internal hole of the fixing member 87 provided in the guide tube 21. The fixing protrusion 88 of the capsule-type endoscope 80 has a general cylinder shape, and is disposed so as to have predetermined engagement as to the internal hole formed in the fixing member 87 of the guide tube 21.

The fixing protrusion 88 of the capsule-type endoscope 80 is configured so as to be integrally fixed with a fixing screw 89 provided in the fixing member 87 in a state of being inserted into the fixing member 87 of the guide tube 21, as illustrated in Fig. 58.

Note that an arrangement may be made wherein a balloon 90 which is expanded and deflated is provided at the tip side of the guide tube 21 without attaching the tip guide member to the tip side of the guide tube 21, as illustrated in Fig. 59.

A hollow collet 91 having a general cylindrical shape is fixed to the tip portion of the guide tube 21. A tube 92 for sending gas to the inside of the balloon 90 or absorbing the air from the inside of the balloon 90 is communicated with the hollow collet 91. The base portion of the balloon 90 is connected to the outer circumferential face at the tip side of the hollow collet 91 in an airtight manner.

The other end portion of the tube 92 is connected in an airtight manner to a collet 93 including a packing 95 at inside thereof. The base opening portion of this collet 105 is connected to a supplied-air suction tube path 94 to be connected to an unshown supplied-air suction pump. The connected portion between the supplied-air suction tube path 94 and the collet 93 is secured with airtightness by the packing 95 provided at the inside of the collet 93. Accordingly, between the inside of the balloon 90 and the supplied-air suction pump is communicated with in an airtight manner.

The gas air-supplied from the supplied-air suction pump, e.g., the air or the like passes through a supplied-air suction tube path 94, collet 93, tube 92, and hollow collet 91, and is supplied to the inside of the balloon 90. Thus, the inside of the balloon 90 is filled with the air, and the balloon 90 is expanded in a general spherical shape by air pressure thereof. A general spherical-shaped guide face is formed on the balloon 90 at the time of being expanded. The guide face is flexible, and also smooth. Note that upon the air at the inside of the balloon 90 being suctioned by the supplied-air suction pump, the balloon 90 is deflated.

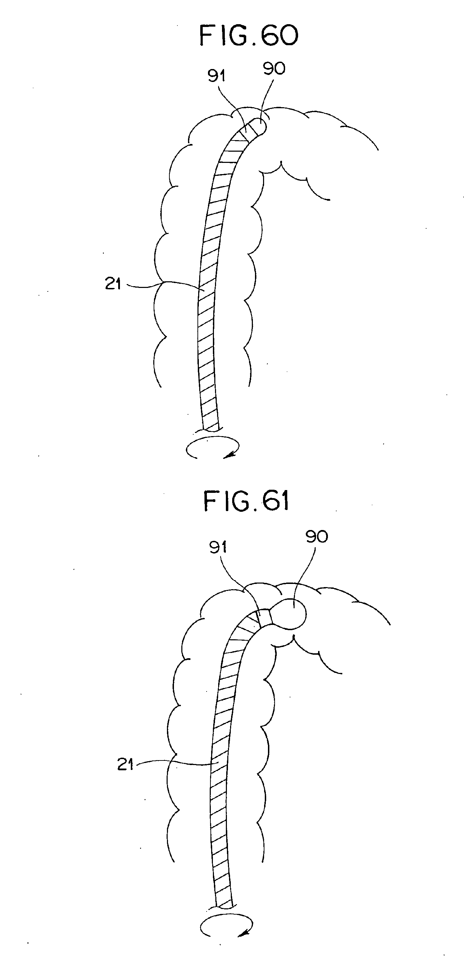

As illustrated in Fig. 60, upon the guide tube 21 reaching the inside of a lumen, e.g., a bending portion of the large intestine, the tip face of the balloon 90 provided at tip thereof is touched with a fold of the intestinal wall. At this time, the air is supplied to the inside of the balloon 90 from the supplied-air suction pump. Then, as illustrated in Fig. 61, the balloon 90 is expanded. The balloon 90 is expanded in a general spherical shape, and thus, a part of the guide face which is surface thereof comes into contact with a fold of the intestinal wall, and the tip portion of the guide tube 21 is bent so as to follow the curve of the intestinal wall. Further, the guide tube 21 advances toward a deep portion of the large intestine by the guide face of the balloon 90 smoothly passing over the folds of the intestinal wall using propulsion thereof.

Thus, the expanded balloon has excellent flexibility, smoothly passes over the folds of the intestinal wall, and the guide tube is inserted into a deep portion of the large intestine, whereby the inner wall of a lumen can be prevented from receiving excessive load from the balloon in a sure manner.

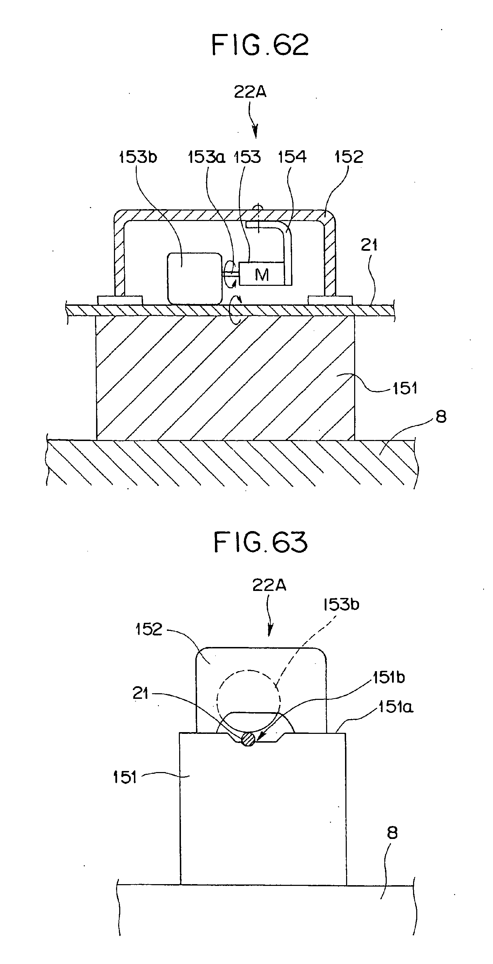

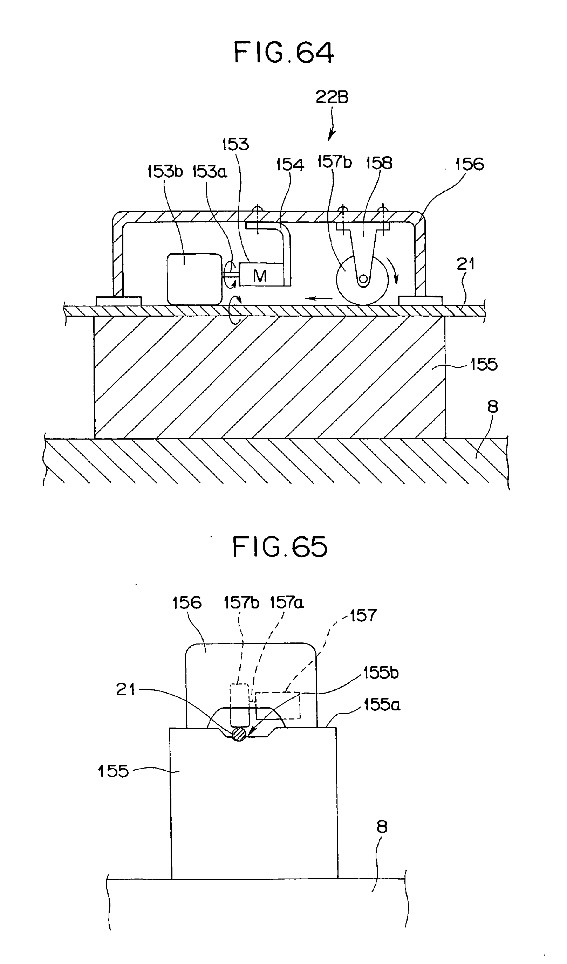

Note that the above embodiments employ a configuration wherein the base side end portion serving as a one end portion of the guide tube 21 is attached to the guide-tube fixing unit 24 fixed to the motor shaft 23a of the motor 23, and the guide tube 21 is rotated, but the configuration of the guide-tube rotating device 22 is not restricted to the above embodiments, e.g., a guide-tube rotating device 22A such as illustrated in Fig. 62 and Fig. 63, or a guide-tube rotating device 22B such as illustrated in Fig. 64 and Fig. 65, or the like may be employed.

The guide-tube rotating device 22A illustrated in Fig. 62 and Fig. 63 comprises a device main body portion 151, a device cover portion 152, and a guide-tube rotating motor (hereinafter, abbreviated as rotating motor) 153. With this guide-tube rotating device 22A, a guide-member disposing groove (hereinafter, abbreviated as groove) 151b where the guide tube 21 is disposed is formed at a predetermined position of a top planar 151a of the device main body portion 151.

The rotating motor 153 is a motor for rotating the guide tube 21 disposed in the groove 151b in the axial rotating direction. A rotating roller 153b having predetermined elastic force which is a rotator is fixedly provided in the motor shaft 153a of the rotating motor 153. The rotating roller 153b is disposed in a state touching the guide tube 21 by predetermined pressure force. Thus, the rotational driving force of the rotating motor 153 is propagated to the guide tube 21, and the guide tube 21 is rotated.

The rotating motor 153 is attached to a predetermined position of the device cover portion 152 by an L-shaped attachment tool 154 for example. The motor shaft 153a of the rotating motor 153 attached to the device cover portion 152 by the attachment tool 154 is in parallel with the top planar 151a of the device main body portion 151, and also is disposed with the positional relation in parallel with the groove 151b. Accordingly, the rotating roller 153b is rotated in a predetermined direction by driving the rotating motor 153, whereby the guide tube 21 can be rotated in the axial rotating direction.

On the other hand, the guide-tube rotating device 22B illustrated in Fig. 64 and Fig. 65 comprises a device main body portion 155, a device cover portion 156, a rotating motor (hereinafter, abbreviated as a first motor) 153, and a guide-tube feeding motor (hereinafter, abbreviated as a second motor) 157. A guide-tube disposing groove (hereinafter, abbreviated as groove) 155b where the guide tube 21 is disposed is formed at a predetermined position of the top planar 155a of the device main body portion 155.

The first motor 153 is a motor for rotating the guide tube 21 disposed in the groove 155b in the axial rotating direction. The rotating roller 153b is fixedly provided in the motor shaft 153a of the first motor 153. The rotating roller 153b has predetermined elastic force, and touches the guide tube 21 with predetermined pressing force. Thus, the rotational driving force of the first motor 153 is propagated to the guide tube 21 via the rotating roller 153b, and the guide tube 21 is rotated.

On the other hand, the second motor 157 is a motor for subjecting the guide tube 21 disposed in the groove 155b to linear-progression movement (hereinafter, also referred to as straight movement) in the longitudinal direction of the guide tube at predetermined speed. A straight movement roller 157b is fixedly provided in the motor shaft 157a of the second motor 157. The straight movement roller 157b has predetermined elastic force, and touches the guide tube 21 with predetermined pressing force. Thus, the rotational driving force of the second motor 157 is propagated to the guide tube 21 via the straight movement roller 157b, and the guide tube 21 is subjected to straight movement.

The first motor 153 is attached to a predetermined position of the device cover portion 156 by a first motor attachment tool 154. The second motor 157 is attached to a predetermined position of the device cover portion 156 by a second motor attachment tool 158.

The motor shaft 153a of the first motor 153 attached to the device cover portion 156 by the first attachment tool 154 is in parallel with the top planar 155a of the device main body portion 155, and also is disposed with the positional relation in parallel with the groove 155b. On the other hand, the motor shaft 157a of the second motor 157 attached to the device cover portion 156 by the second attachment tool 158 is in parallel with the top planar 155a of the device main body portion 155, and also is disposed with the positional relation orthogonal to the groove 155b.

Accordingly, the rotating roller 153b is rotated in a predetermined direction by driving the first motor 153, whereby the guide tube 21 can be rotated in the axial rotating direction. Also, the straight movement roller 157b is rotated in a predetermined direction by driving the second motor 157, whereby the guide tube 21 can be subjected to straight movement in the longitudinal direction.

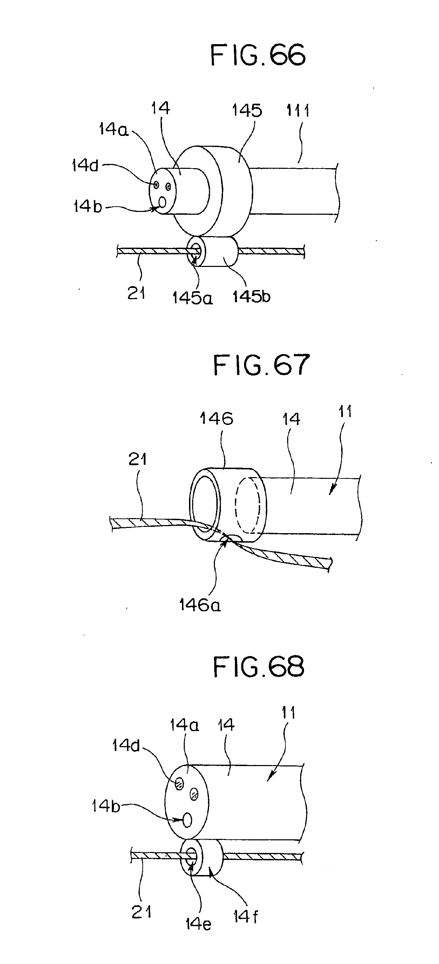

Also, with the above embodiments, following inserting the guide tube 21 into a lumen, the surgeon inserts the base side from the half-way portion of the guide tube 21 into the treatment-tool insertion channel 11 a provided in the insertion unit 11 of the endoscope 2, and inserts the insertion unit 11 into a deep portion within the large intestine while confirming the observation image of the guide tube 21 on the screen. However, as illustrated in Fig. 66 through Fig. 69, an arrangement may be made wherein the surgeon inserts the insertion unit 11 of the endoscope 2 into a deep portion within a body cavity while observing the guide tube 21.

A method for inserting the insertion unit of the endoscope into a deep portion within a body cavity while observing the guide tube disposed within the body cavity will be described with reference to Fig. 66 through Fig. 69.

In Fig. 66, an arrangement is made wherein a guide-tube insertion assisting tool 145 is mounted on the tip rigid portion 14 of the insertion unit 11. The guide-tube insertion assisting tool 145 is provided with a guide-tube insertion salient portion 145b including a guide-tube insertion hole 145a. The guide-tube insertion hole 145a is configured such that the guide tube 21 is inserted therein.

That is to say, with the configuration of the present drawing, following inserting the guide tube 21 into a body cavity, the surgeon inserts the base side from the half-way portion of the guide tube 21 not into the treatment-tool insertion channel 11a provided in the insertion unit 11 of the endoscope 2, but into the guide-tube insertion hole 145a provided in the guide-tube insertion assisting tool 145 mounted on the tip rigid portion 14.

Subsequently, as with the above embodiments, the surgeon makes the endoscope 2 observable to insert the insertion unit 11 into the large intestine, and inserts the tip rigid portion 14 of the insertion unit 11 on which the guide-tube insertion assisting tool 145 is mounted into the large intestine from the anus 71. Then, an endoscope image including the image of the guide tube 21 is displayed on the screen of the monitor 6. Here, the surgeon performs an operation for bending the bending portion 15, an operation for twisting the insertion unit 11, or the like while confirming the extending direction of the guide tube 21 on the screen of the monitor 6, and inserts the tip rigid portion 14 of the insertion unit 11 toward a deep portion within the large intestine. Thus, in addition to the same operations and advantages as that in the above embodiments, the surgeon can be released from botheration for inserting the guide tube 21 into the treatment-tool insertion channel 11a.

Note that as illustrated in Fig. 67, even if a tip cap 146 in which a guide-tube insertion hole 146a is provided is mounted on the tip rigid portion 14 of the insertion unit 11 instead of the guide-tube insertion assisting tool 145, the same operations and advantages can be obtained. Also, as illustrated in Fig. 68, even if a guide-tube insertion salient portion 14f where a guide-tube insertion hole 14e is formed is provided in the tip rigid portion 14 of the insertion unit 11 instead of mounting the guide-tube insertion assisting tool 145 or the tip cap 146 on the insertion unit 11, the same operations and advantages can be obtained.

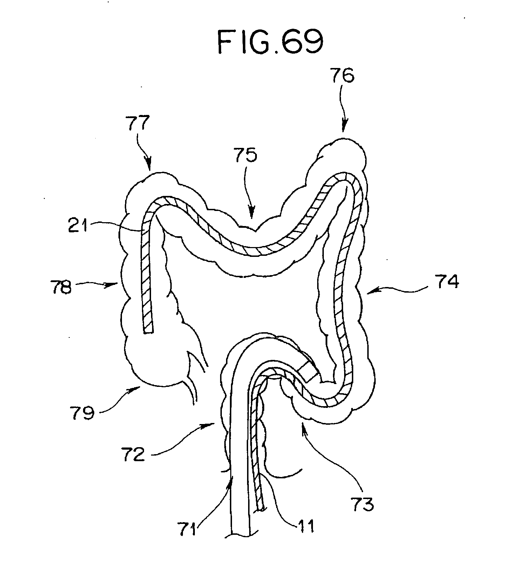

Also, in Fig. 69, the surgeon inserts the insertion unit 11 toward a deep portion within a lumen in a state in which the guide tube 21 is disposed within a body cavity without inserting the guide tube 21 inserted into a body cavity into the treatment-tool insertion channel 11 a provided in the insertion unit 11 of the endoscope 2, or into the guide-tube insertion holes 145a, 146a, or 14e.

That is to say, the surgeon inserts the insertion unit 11 of the endoscope 2 into the large intestine from the anus 71 in a state in which the guide tube 21 is inserted into the large intestine, and displays the image of the guide tube 21 on the screen of the monitor 6. Subsequently, the surgeon inserts the tip rigid portion 14 of the insertion unit 11 into a deep portion within the large intestine while confirming the extending direction of the guide tube 21 inserted into the body cavity on the screen, and while performing an operation for bending the bending portion 15, an operation for twisting the insertion unit 11, or the like. Thus, the surgeon is released from botheration for inserting the guide tube 21 into the treatment-tool insertion channel 11 a, or the guide-tube insertion holes 145a, 146a, and 14e.

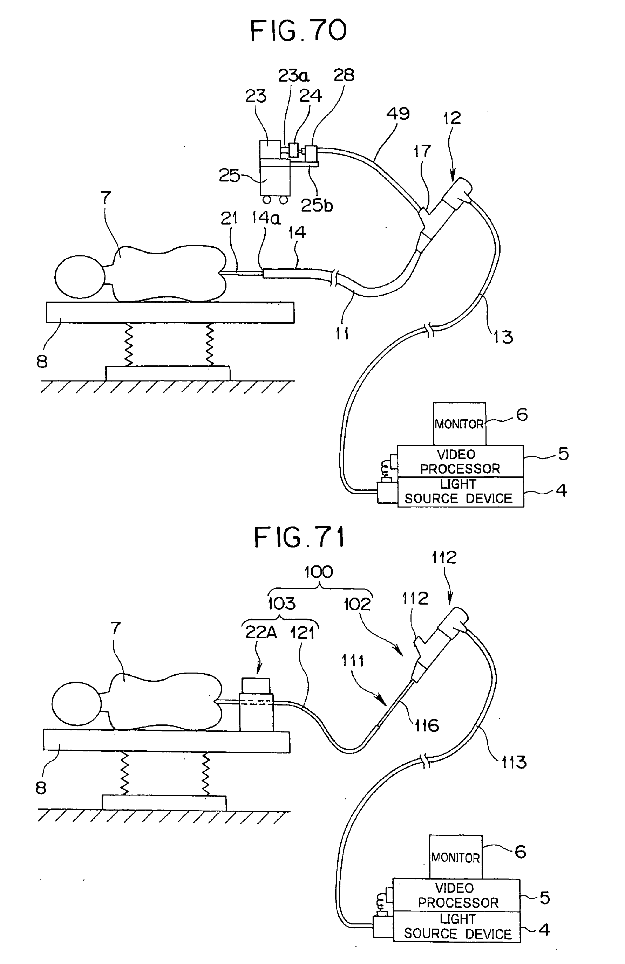

Also, as illustrated in Fig. 70, an arrangement may be made wherein the surgeon attaches the base portion of the guide tube 21 to the guide-tube fixing unit 24 beforehand, inserts the tip side of the guide tube 21 into the treatment-tool insertion channel 11a from the treatment-tool entrance 17, and protrudes this from the tip opening 14b. Note that reference numeral 49 is a protective tube, and is disposed between the protective-tube holding member 28 and the treatment-tool entrance 17. The other configurations are the same as the insertion device 1 illustrated in Fig. 1, the same members are denoted with the same reference numerals, and description thereof will be omitted.

Operations of the insertion device 1 according to the present embodiment will be described.

As described above, a procedure for inserting the guide tube 21 protruded from the tip opening 14b into the large intestine will be described.

First, the surgeon grips the tip side portion of the guide tube 21 protruded from the tip opening of the treatment-tool insertion channel 11 a provided in the insertion unit 11. Subsequently, the surgeon inserts the tip portion of the guide tube 21 into the large intestine from the anus of the patient 7 lying on the bed 8. Subsequently, the surgeon causes the motor 23 to be a rotational driving state, as described above. Thus, the guide tube 21 in a rotating state smoothly advances along each wall of the rectum 72, sigmoid colon portion 73, descending colon portion 74, transverse colon portion 75, and ascending colon portion 78. As a result of this, the tip portion of the guide tube 21 reaches near the appendix 79 which is a target portion for example without changing the course of the large intestine.

Upon determining that the tip portion of the guide tube 21 has reached near the appendix 79, the surgeon subsequently inserts the tip rigid portion 14 of the insertion unit 11 of the endoscope 2 into the large intestine from the anus 17. That is to say, following inserting the guide tube 21 into the large intestine, the surgeon subsequently inserts the insertion unit 11 of the endoscope 2 into the large intestine. Subsequently, in the same way as described above, the surgeon performs an operation for bending the bending portion 15, an operation for twisting the insertion unit 11, or the like while confirming the extending direction of the guide tube 21 to be displayed on the screen of the monitor 6, and inserts the tip rigid portion 14 of the insertion unit 11 into a deep portion within the large intestine. At this time, the surgeon can smoothly insert the tip rigid portion 14 of the insertion unit 11 into near the appendix 79 without mistaking the insertion direction.

Thus, the surgeon puts the guide tube in a state of having been inserted into the treatment-tool insertion channel provided in the insertion unit beforehand, and first, inserts only the guide tube into a target portion within the large intestine. Thus, following inserting the guide tube into the target portion, the surgeon can omit a procedure for inserting the guide tube into the treatment-tool insertion channel, and can insert the insertion unit of the endoscope into a body cavity. Accordingly, time period between the start of inserting the guide tube and the start of inserting the endoscope can be reduced. Note that the other operations and advantages are the same as those in the above embodiments.

Description will be made regarding a sixth embodiment of the present invention with reference to Fig. 71 through Fig. 78.

As illustrated in Fig. 7, an insertion device 100 according to the present embodiment principally comprises an endoscope 102, and an endoscope insertion assisting tool 103.

The endoscope 102 comprises an insertion unit 111, an operating unit 112, and a universal cord 113. The operating unit 112 is provided at the base side of the insertion unit 111. The universal cord 113 extends from the side portion of the operating unit 112.

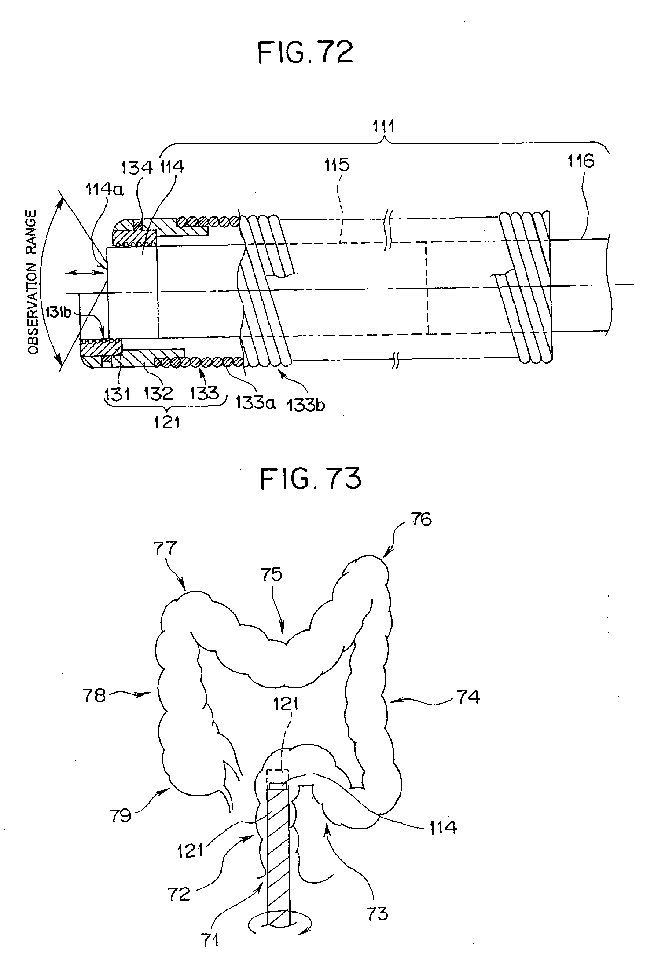

The insertion unit 111 is configured so as to consecutively have a tip rigid portion (see reference numeral 114 in Fig. 72), a bending portion (see reference numeral 115 in Fig. 72), and a flexible tube portion 116 in order from the tip side thereof. The bending portion 115 is configured so as to bend in the vertical and horizontal directions for example. The flexible tube portion 116 has flexibility. The operating unit is provided with a treatment-tool entrance 117. The treatment-tool entrance 117 is connected to a treatment-tool insertion channel (not shown) for inserting a treatment tool provided within the insertion unit 111.

The endoscope 102 comprises a light source device 4, a video processor 5, and a monitor 6, which serve as external devices. The light source device 4 supplies illumination light to the endoscope 102. The video processor 5 includes a signal processing circuit, supplies a driving signal for driving an unshown image capturing device provided in the endoscope 102, and also generates a picture signal from the electric signal photoelectric-converted and transmitted by the image capturing device to output this to the monitor 6. An endoscope image is displayed on the screen of the monitor 6 in response to the picture signal output from the video processor 5.

The endoscope insertion assisting tool 103 is an insertion assisting member, and principally comprises a guide tube 121, and the above guide-tube rotating device 22A, for example. The guide-tube rotating device 22A is installed on the bed 8 where the patient 7 is lying, for example.

As illustrated in Fig. 72, the insertion unit 111 of the endoscope 102 is disposed in the inner hole which is the internal space of the guide tube 121. Specifically, the guide tube 121 comprises a rotational moving member 131, an assisting-tool main body portion 132, and a spiral tube portion 133.

The rotational moving member 131 comprises a tube-shaped rotating main body portion 131a, and multiple bearings 131b to be disposed at the inner circumferential face side of this rotating main body portion 131a. The rotational moving member 131 is a mounting unit, and a tip rigid portion 114 making up the insertion unit 111 of the endoscope 102 is disposed at the inner circumferential face side of the rotational moving member 131, for example. In this placement state, the rotational moving member 131 can move rotationally as to the tip rigid portion 114.

On the other hand, the spiral tube portion 133 is formed by winding a metal wire 133a made of stainless steel having a predetermined diameter dimension in a spiral shape so as to have predetermined flexibility, for example. A spiral-shaped portion 133b which is formed with the surface of the metal wire 133a is provided on the outer surface of the spiral tube portion 133. Note that an arrangement may be made wherein multiple (e.g., quadruple) spirals of the metal wire are wound to form the spiral-shaped portion 133b. In this case, when winding a metal wire spirally, various types of properties of the guide tube 21 can be set by improving density between metal wires, or changing the angle of spirals.

The rotational moving member 131 is integrally fixed at a predetermined position of the tip side inner circumferential face of the assisting tool main body portion 132 using a fixing screw 134 for example. Also, one end portion of the spiral tube portion 133 is integrally fixed at the base side outer circumferential face of the assisting tool main body portion 132 by bobbin-winding adhesion (not shown) for example. That is to say, the insertion unit 111 of the endoscope 102 is disposed in a loosely fit manner as to the assisting tool main body portion 132 and the spiral tube portion 133. In this placement state, the guide tube 121 is a so-called overtube for covering the insertion unit 111 of the endoscope 102, so that this overtube serves as the insertion unit of the endoscope 102.

Accordingly, the guide tube 121 integrally configured of the rotational moving member 131, the assisting tool main body portion 132, and the spiral tube portion 133 can move rotationally as to the insertion unit 111, and also can advance and retreat such as illustrated with the arrow.

Note that the tip side of the assisting tool main body portion 132 is subjected to chamfering process for preventing the body wall and mucous membrane within a body cavity from being scratched, and the like.

Operations of the insertion device 100 thus configured will be described.

A preparatory procedure up to inserting the insertion unit 111 of the endoscope 102 into the large intestine will be described.

When inserting the insertion unit 111 of the endoscope 102 into the appendix of the large intestine for example, the staff first prepares for the guide tube 121 including the rotational moving member 131 having a predetermined inner diameter dimension. Next, the staff inserts the insertion unit 111 of the endoscope 102 into the inner hole of the guide tube 121. Subsequently, the staff protrudes the tip rigid portion 114 of the insertion unit 111 only for a predetermined amount as to the rotational moving member 131 such as illustrated at the upper side from the center line in Fig. 72 for example, or disposes this in a general face matching state. In this placement state of the endoscope, the observation field of view of the endoscope 102 is prevented from being hindered by the guide tube 121A.

Next, the staff disposes the insertion unit 111 in a state in which the guide tube 121 is covered in the guide-tube rotating device 22A. At this time, the staff disposes the tip face of the guide tube 121 in the groove 151b in a state of being protruded from the tip side end face of the device main body portion 151 only for a predetermined amount for example. Also, the staff turns on the light source device 4, video processor 5, and monitor 6. Thus, the preparation for inserting the insertion unit 111 toward a deep portion of the large intestine in a state in which the guide tube 121 is mounted on the insertion unit 111 is completed.

A procedure example for inserting the insertion unit 111 of the endoscope 102 mounting the guide tube 121 into the large intestine will be described.

As illustrated in the above Fig. 71, a surgeon (not shown) grips the side toward the surgeon of the insertion unit 111, and inserts the tip side portion of the guide tube 121 in a state in which the guide tube 121 is covered into the large intestine from the anus of the patient 7 lying on the bed 8. Then, an observation image within the large intestine cast by the illumination light emitted from the illumination window (not shown) provided on the tip face of the tip rigid portion 114 is formed on the image capturing face of the image capturing device through an observation window 114b making up an observation unit 114a, and an endoscope image is displayed on the screen of the monitor 6. At this time, the spiral-shaped portion 133b formed on the outer surface of the spiral tube portion 133 making up the guide tube 121 comes into contact with the intestinal wall.

In this contact state, the contact state between the spiral-shaped portion 133b formed on the spiral tube portion 133 and the intestinal wall is the relation between a male screw and a female screw. Here, the surgeon rotates and drives the rotating motor 153 of the guide-tube rotating device 22A. The guide tube 121 is rotated in the axial rotating direction such as illustrated with the arrow in Fig. 73 by the rotating roller 153b being rotated. That is to say, the guide tube 121 is in a state of being rotated in the axial rotating direction as to the insertion unit 111 by the rotational driving force of the rotating motor 153 being propagated to the spiral tube portion 133 via the rotating roller 153b.

Propulsion for advancing the guide tube 121 is generated at the contact portion between the spiral-shaped portion 133b and the intestinal wall, such as a male screw moving as to a female screw, by the guide tube 121 being rotated. Then, the guide tube 121 is advanced along the wall of the rectum 72 by the propulsion, and is advanced toward a deep portion within the large intestine such as illustrated with the dashed line in Fig. 73, for example. At this time, as illustrated in the lower side from the center line in Fig. 72, the tip side portion of the guide tube 121 is in a state of being protruded from the tip face of the tip rigid portion 114. Thus, a part of an observation range of the observation optical system provided on the tip face of the tip rigid portion 114 is interrupted by the rotational moving member 131 of the guide tube 121.

At this time, an endoscope image in which a part of the image of the large intestine is interrupted by the guide tube 121 is displayed on the screen of the monitor 6 which the surgeon is observing. That is to say, when confirming the image of the guide tube 121 at a part of the endoscope image to be displayed on the screen, the surgeon determines that the guide tube 121 has advanced. Subsequently, the surgeon performs a manual operation for inserting the insertion unit 111 for a predetermined amount. Then, the insertion unit 111 is moved forward within the inner hole of the guide tube 121, and thus, the tip rigid portion 114 is in a state of being protruded for a predetermined amount as to the rotational moving member 131, or a general face-matching state again such as illustrated in the upper side from the center line in Fig. 72. At this time, only the endoscope image of the large intestine not including the image of the guide tube 121 is displayed on the screen.

That is to say, the surgeon confirms whether or not there is the image of the guide tube 121 at a part of the endoscope image to be displayed on the screen of the monitor 6, and when the image of the guide tube 121 is displayed, the surgeon determines the amount of protrusion from the position of image thereof, performs an operation for bending the bending portion 115, an operation for twisting the insertion unit 111, or a manual operation for pressing the insertion unit 111 forward, and inserts the insertion unit 111 toward a deep portion within the large intestine.

Subsequently, when the guide tube 121 reaches a bending portion such as near the sigmoid colon portion 73 or the like such as illustrated in the dashed line in Fig. 73, the contact length between the spiral-shaped portion 133b formed on the outer surface of the guide tube 121 and the intestinal wall is long, so the guide tube 121 which is rotating smoothly advances within the sigmoid colon portion 73 which is bent and also is where mobility is good by the propulsion. At this time, the surgeon observes the endoscope image to be displayed on the screen of the monitor 6. Subsequently, upon confirming the image of the guide tube 121 interrupting the endoscope image, the surgeon performs a manual operation as appropriate, and moves the tip rigid portion 114 of the insertion unit 111 not as to the intestinal wall but as to the inside of the inner hole of the guide tube 121.

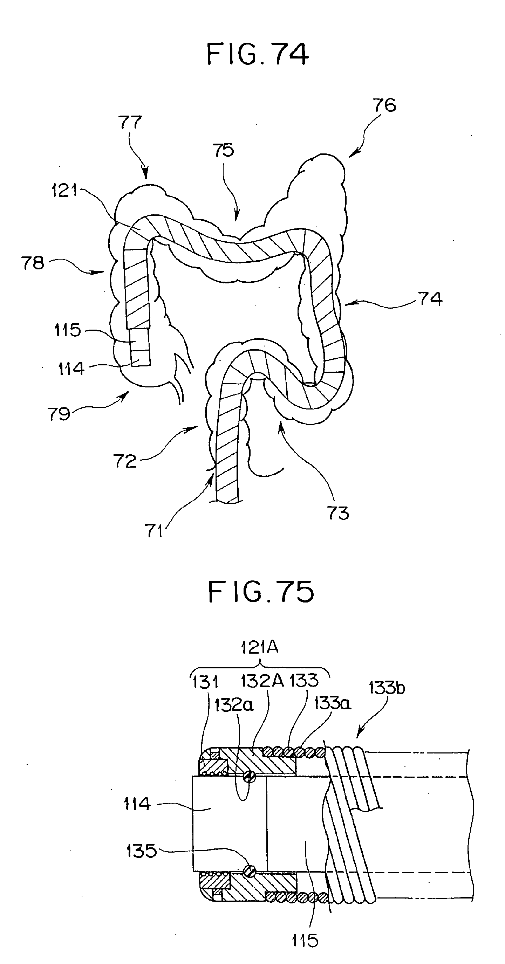

That is to say, the surgeon repeatedly performs movement of the guide tube 121 within the large intestine by the propulsion, and movement for advancing the insertion unit 111 to the inside of the inner hole of the guide tube 121 by performing a pressing forward operation. Thus, the guide tube 121 and the insertion unit 111 pass through the sigmoid colon portion 73, following which smoothly advance along the walls of a bending portion serving as the boundary between the sigmoid colon portion 73 and the descending colon portion 74 where mobility is poor, the splenic flexure 76 serving as the boundary between the descending colon portion 74 and the transverse colon portion 75 where mobility is good, and the hepatic colic flexure 77 serving as the boundary between the transverse colon 75 and the ascending colon portion 78, and as illustrated in Fig. 74, reach near the appendix 79 which is a target portion for example without greatly changing the course of the large intestine.

Upon confirming from the endoscope image to be displayed on the screen that the insertion unit 111 has reached near the appendix 79, the surgeon stops rotation of the rotating motor 153 of the guide-tube rotating device 22A. Thus, the advancement of the guide tube 121 is stopped. Here, the surgeon makes the transition to a retraction operation of the insertion unit 111 to perform endoscopy within the large intestine. At this time, the surgeon performs endoscopy in a state in which the guide tube 121 is mounted on the insertion unit 111, and also the tip rigid portion 114 of the insertion unit is protruded only for a predetermined amount, or in a general face-matching state.

Note that an unshown foot switch may be provided in the endoscope insertion assisting tool 103 according to the present embodiment to perform driving control of the rotating motor 153.

Thus, the insertion unit of the endoscope is disposed in the inner hole of the guide member including the spiral tube portion in which the spiral-shaped portion is provided, the insertion unit in a state in which the guide member is mounted is inserted into the large intestine, and the guide member is set to a rotating state, and thus, the guide member is rotated as to the insertion unit, and thus, rotating force thereof is converted into propulsion, and the guide member covering the insertion unit moves as to the large intestine and this insertion unit. Subsequently, when confirming movement of the guide member by the endoscope image which the surgeon is observing, the surgeon moves the insertion unit as to the guide member. Subsequently, the surgeon repeatedly performs movement of the guide member as to the large intestine by the propulsion, and movement of the insertion unit as to the inner hole of the guide member by a manual operation, whereby the surgeon can insert the insertion unit toward a deep portion of the large intestine via the guide member.

Thus, when confirming the image of the guide member to be displayed on the screen of the monitor, the surgeon can perform an appropriate bending operation, and twisting operation, and also smoothly perform insertion of the insertion unit up to a deep portion of a lumen in a short period of time while reducing the amount of a pressing forward operation.

Also, with the endoscope insertion assisting tool made up of the guide member and the guide-member rotating device, the spiral-shaped portion is provided on the outer surface of the spiral tube portion making up the guide member. Thus, in a state in which the insertion unit in a state of being covered with the guide member is inserted into the large intestine, the contact state between the spiral-shaped portion formed on the guide member and the intestinal wall is the so-called relation between a male screw and a female screw. Accordingly, in this contact state, the motor making up the guide-member rotating device is rotated and driven, and thus, the rotating force in the axial rotating direction of the guide member is converted into propulsion such as a male screw moving as to a female screw, whereby the guide member can be smoothly advanced as to the large intestine.

Note that with the above embodiments, an arrangement has been made wherein only the guide member is moved by the propulsion of the guide member, following which the insertion unit is moved as to the inner hole of the guide member, movement of the guide member and movement of the insertion unit is repeatedly performed, and thus the insertion unit is inserted toward a deep portion of the large intestine. However, an arrangement may be made wherein the insertion unit 111 of the endoscope 102, and an guide tube 121 A are configured such as shown in the following, thereby moving the insertion unit 111 to a deep portion within a body cavity by the propulsion of the guide tube 121 A.

Another configuration for inserting the insertion unit 111 of the endoscope 102 into a deep portion within a body cavity will be described with reference to Fig. 75.

As illustrated in the drawing, a guide tube 121 A according to the present embodiment is configured so as to move rotationally at the same position as to the insertion unit 111 of the endoscope 102. That is to say, positioning rotational moving means are provided in the tip rigid portion 114 making up the assisting tool main body portion 132A and the insertion unit 111 for preventing the guide tube 121A from being advanced and retracted as to the insertion unit 111.

The positioning rotational moving means comprises a circumferential groove 132a provided at an inner circumferential face predetermined position of the assisting tool main body portion 132A, an a locking member 135 which is disposed on the outer circumferential face of the tip rigid portion 114, and is fixed in the circumferential groove 132a. The locking member 135 has predetermined elastic force.

When inserting the insertion unit 111 of the endoscope 102 into the inner hole of the guide tube 121 A, the assisting tool main body portion 132A of the guide tube 121A is externally fitted and disposed in a predetermined position of the tip rigid portion 114 in the teeth of the pressing force of the locking member 135. Thus, the locking member 135 provided in the tip rigid portion 14 is fixed in the circumferential groove 132a of the assisting tool main body portion 132A. Then, the guide tube 121A can be moved rotationally without being advanced and retracted as to the insertion unit 111. In this placement state of the endoscope, the tip face of the endoscope 102 is disposed more forward side than the assisting tool main body portion 132A so as to protrude from the tip face of the assisting tool main body portion 132A. Accordingly, the observation field of view of the endoscope 102 is prevented from being hindered by the guide tube 121A in a sure manner.

Subsequently, for example, as illustrated in Fig. 73, in a state in which the spiral-shaped portion 133b formed on the outer surface of the spiral tube portion 133 is in contact with the intestinal wall, when the rotating motor 153 is rotated and driven to generate propulsion at the guide tube 121A, the guide tube 121A starts advancement along the wall of the rectum 72 by the propulsion. At this time, the locking member 135 provided in the tip rigid portion 114 is fixed in the circumferential groove 132a of the assisting tool main body portion 132A making up the guide tube 121A, so that the insertion unit 111 is also integrally advanced along with movement of the guide tube 121A. That is to say, the guide tube 121 A is moved toward a deep portion within the large intestine, whereby the insertion unit 111 locked and disposed in the inner hole of this guide tube 121A is also moved toward a deep portion within the large intestine in the same way.

Thus, while the circumference groove is formed in the assisting tool main body portion making up the guide tube, the locking member is provided in the tip rigid portion making up the insertion unit of the endoscope, whereby the guide tube is configured so as to be moved rotationally without being advanced and retracted as to the insertion unit. Thus, in a state in which the guide tube is rotating to generate propulsion, the insertion unit of the endoscope can be advanced and retracted as to the large intestine along with advancement and retreat of the guide tube.

Note that with the present embodiment, the positioning rotational moving means comprises the circumferential groove 132a provided at an inner circumferential face predetermined position of the assisting tool main body portion 132A, the locking member 135 which is disposed on the outer circumferential face of the tip rigid portion 114, and is fixed in the circumferential groove 132a. However, the positioning rotational moving means is not restricted to this, so an arrangement may be made wherein the circumferential groove is provided on the tip rigid portion side, and the locking member is provided at the inner circumferential face side of the assisting tool main body portion 132A.

Also, with the present embodiment, the rotational direction of the motor is such that the guide member is rotated in one direction of the longitudinal axial rotating direction of the guide member, but the motor may be a type capable of optionally left-rotating or right-rotating the guide member.

Also, with the present embodiment, insertion of the insertion unit 111 up to a deep portion is realized by the surgeon repeatedly performing movement of the guide tube 121 as to the large intestine, and movement of the insertion unit 111 as to the inner hole of the guide tube 121 by a manual operation while confirming the endoscope image on the screen. However, an arrangement may be made wherein the surgeon inserts the insertion unit 111 of the endoscope 102 up to a deep portion within a body cavity via the guide tube 121 such as shown in the following.

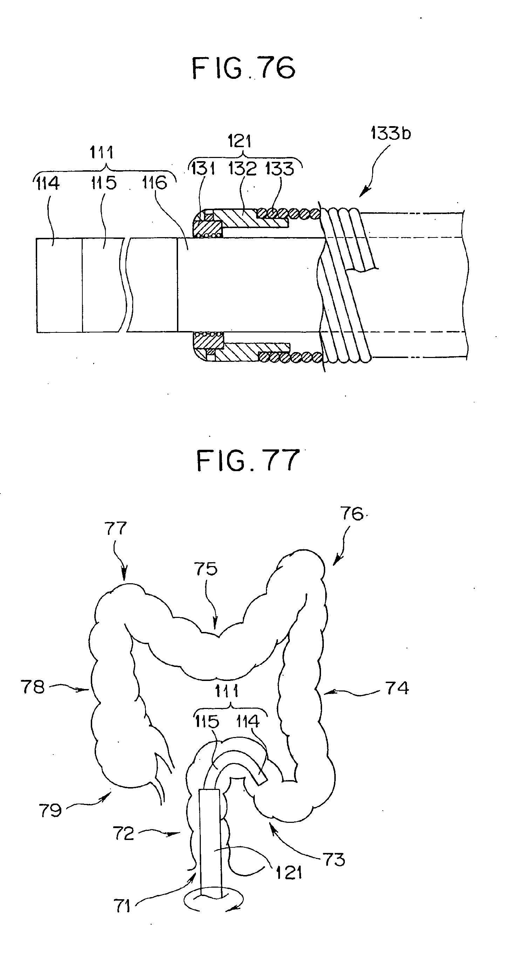

Yet another configuration for inserting the insertion unit 111 of the endoscope 102 up to a deep portion within a body cavity will be described with reference to Fig. 76 through Fig. 78.

As illustrated in Fig. 76, with the present embodiment, the tip face of the guide tube 121 is disposed on a flexible tube 116 making up the insertion unit 111. That is to say, the bending portion 115 of the endoscope 102 is disposed more tip side than the tip face of the guide tube 121.

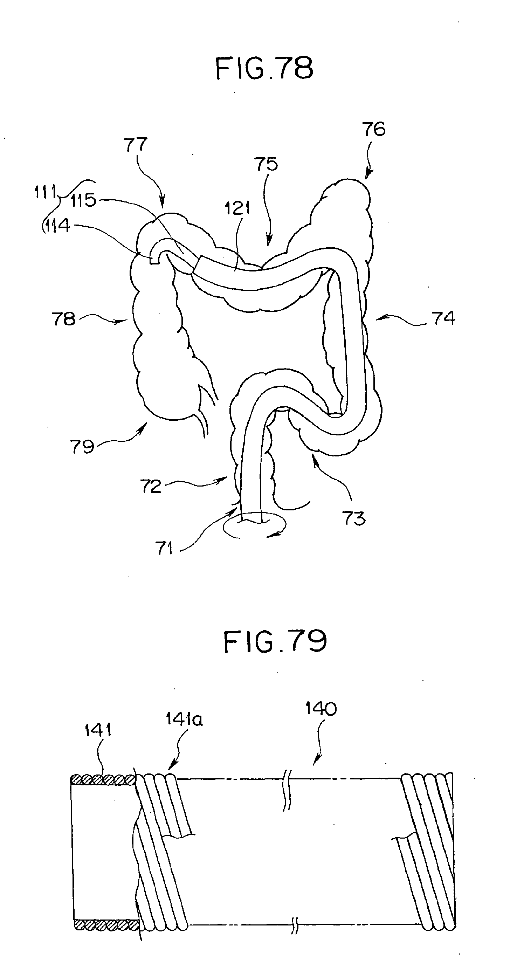

When inserting the insertion unit 111 where the guide tube 121 is disposed into the large intestine, the surgeon makes the endoscope 102 into an observable state. Subsequently, the surgeon inserts the insertion unit 111 protruding from the tip face of the guide tube 121 into the large intestine from the anus 71. Then, the endoscope image captured by the endoscope 102 is displayed on the screen of the monitor 6. Here, the surgeon inserts the insertion unit 111 toward a deep portion within the large intestine while observing the endoscope image. Then, the guide tube 121 is also inserted into the large intestine from the anus 71. Here, the surgeon moves the insertion unit 111 toward a deep portion within the large intestine using the propulsion of the guide tube 121 while observing the endoscope image, as illustrated in Fig. 77 and Fig. 78.

That is to say, the surgeon determines the insertion direction from the endoscope image displayed on the screen of the monitor 6, and keeps his/her mind on a manual operation such as an operation for appropriately bending the bending portion 115, or an operation for twisting the insertion unit 111, or the like. The manual operation for moving the insertion unit 111 toward a deep portion depends on the propulsion of the guide tube 121. Thus, the surgeon can smoothly perform insertion of the insertion unit up to a luminal deep portion.

A seventh embodiment of the present invention will be described with reference to Fig. 79 through Fig. 83.

As illustrated in Fig. 79, a guide tube 140 serving as an insertion-unit guide member according to the present embodiment is a spiral tube in which ease of insertion into a body cavity is considered, e.g., a tube which is formed so as to have predetermined flexibility by winding a metal wire 141 made of stainless steel having a predetermined diameter dimension in a spiral shape. Accordingly, the outer surface of the guide tube 140 is provided with a spiral-shaped portion 141a which the surface of the metal wire 141 forms. Note that the inner diameter of the guide tube 140 is set so as to allow insertion of the insertion unit 111 of the above endoscope 102.

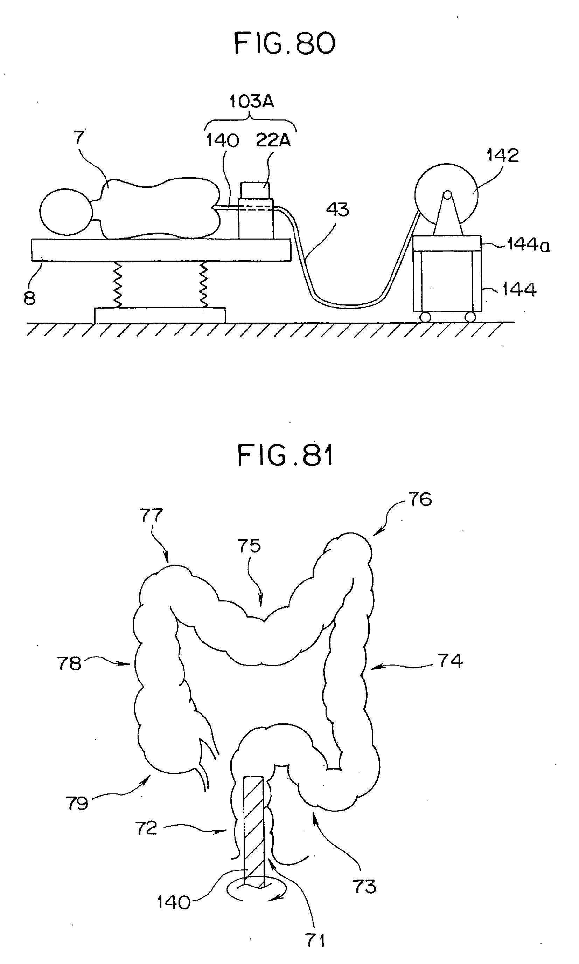

As illustrated in Fig. 80, an endoscope insertion assisting tool 103A according to the present embodiment comprises a guide tube 140, and a guide-tube rotating device 22A. With the present embodiment, the guide tube 140 is disposed in a loosely fit manner so as to be rotatable and slidable within a protective tube 143 wound around a drum 142. The drum 142 is installed on a pedestal 144a of a drum cart 144, for example. The other configurations are the same as those in the sixth embodiment, so the same members are denoted with the same reference numerals, and description thereof will be omitted.

A procedure for inserting the guide tube 140 into the large intestine will be described.

First, as illustrated in Fig. 80, a surgeon (not shown) grips the tip side portion of the guide tube 140, and inserts the tip portion of the guide tube 140 into the large intestine from the anus of the patient 7 lying on the bed 8. Then, the spiral-shaped portion 141 a formed on the outer surface of the guide tube 140 comes into contact with the intestinal wall. At this time, the contact state between the spiral-shaped portion 141a formed on the guide tube 140 and the intestinal wall is the relation between a male screw and a female screw.

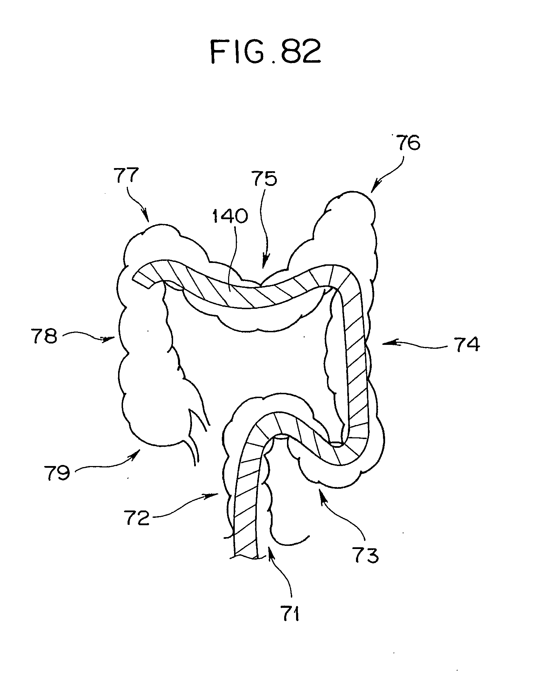

In this contact state, the surgeon makes the rotating motor 153 of the guide-tube rotating device 22 a rotational driving state. Then, as illustrated with the arrow in Fig. 81, the spiral-shaped portion 141a of the guide tube 140 is in a state of being rotated in the axial rotating direction such as moving from the base side to the tip side.

Thus, propulsion for advancing the guide tube 140, such as a male screw moving as to a female screw, is generated at the contact portion between the rotated spiral-shaped portion 141 a of the guide tube 140 and the intestinal wall. Then, the guide tube 140 is advancing toward a deep portion within the large intestine by the propulsion. At this time, the surgeon may perform a manual operation such as pressing the griped guide tube 140 forward.

Then, the guide tube 140 inserted from the anus 71 is advancing toward the sigmoid colon portion 73 from the rectum 72 by the propulsion and the manual operation. Subsequently, as illustrated in Fig. 81, the guide tube 140 reaches near the sigmoid colon portion 73. At this time, the contact length between the spiral-shaped portion 141a formed on the outer surface of the guide tube 140 and the intestinal wall is long, so that the rotated guide tube 140 is smoothly advancing within the sigmoid colon portion 73 which is bent, and where mobility is good.

Subsequently, the guide tube 140 in a rotated state passes through the sigmoid colon portion 73, and is smoothly advancing along the walls of a bending portion which is the boundary between the sigmoid colon portion 73 and the descending colon portion 74 where mobility is poor, the splenic flexure 76 which is the boundary between the descending colon portion 74 and the transverse colon portion 75 where mobility is good, and the hepatic colic flexure 77 which is the boundary between the transverse colon portion 75 and the ascending colon portion 78. Then, the guide tube reaches, for example, near the hepatic colic flexure 77 of the ascending colon portion 78 which is a target portion without changing the course of the large intestine, as illustrated in Fig. 82.

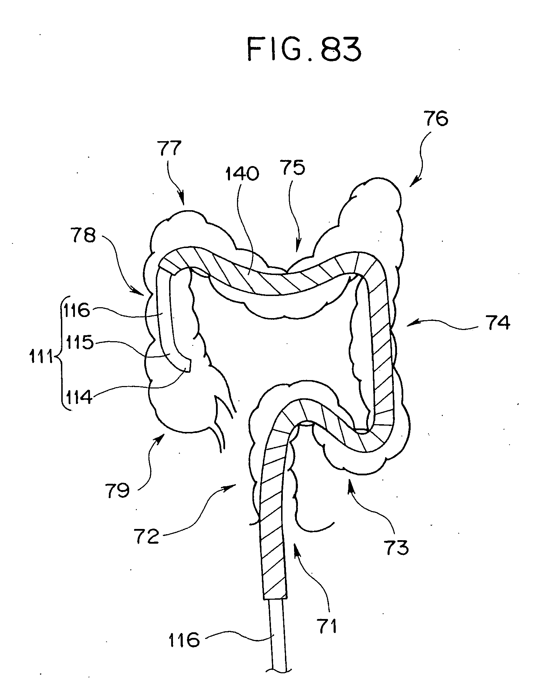

When the surgeon determines that the guide tube has reached the target portion, the surgeon subsequently inserts the insertion unit 111 of the endoscope 102 into the inner hole of the guide tube 140 from the base side opening of the guide tube 140 protruding outside from the anus 71. Then, the surgeon performs insertion of the insertion unit 111 while confirming the extending direction of the guide tube 140 on the screen of the monitor 6, and while performing an operation for bending the bending portion 115, an operation for twisting the insertion unit 111, or the like. Then, as illustrated in Fig. 83, the insertion unit 111 protrudes from the tip side opening of the guide tube 140 positioned within the large intestine, and the tip rigid portion 114 is disposed near the appendix 79.

With the present embodiment, the insertion unit 111 advances within the inner hole of the guide tube 140 inserted into the large intestine, and is guided to a deep portion of the large intestine. Accordingly, the surgeon can smoothly insert the insertion unit 111 into near the appendix 79 without mistaking the direction to be inserted while extremely reducing the amount of insertion force. Subsequently, when confirming on the screen of the monitor 6 that the insertion unit 111 has reached near the appendix 79 which is the target portion, the surgeon subsequently performs endoscopy within the large intestine.

Thus, following inserting the guide member up to the target portion within the large intestine beforehand without combining the guide member and the insertion unit of the endoscope, the surgeon inserts the insertion unit of the endoscope into the inner hole of the guide member from the base side opening of the guide member disposed outside the body. Thus, the surgeon can insert the insertion unit of the endoscope into the target portion with the small amount of insertion force while performing an appropriate bending operation, or twisting operation without mistaking the insertion direction where the insertion unit is inserted.

Thus, versatility is improved as to a plurality of medical equipment including an insertion unit of which outside diameter differs by inserting medical equipment such as an endoscope or the like into an insertion assisting member. Also, the insertion assisting member is positioned at the outer side of the insertion unit, and accordingly, with medical equipment including a treatment-tool insertion channel, the treatment-tool insertion channel is not used by the insertion assisting member.

Accordingly, with medical equipment such as an endoscope including only one treatment-tool insertion channel, when performing treatment as to a target portion such as an affected portion or the like using a treatment tool, a surgeon can perform treatment at once without performing work such as removing the insertion assisting member from the treatment-tool insertion channel, or the like, whereby treatment readiness can be improved.

Note that as described above, the insertion assisting member for assisting insertion of medical equipment by inserting the medical equipment into the inside thereof is configured so as to be rotated, and the medical equipment is protruded from the tip side of the insertion assisting member, and thus, observation, treatment, or the like of a subject can be prevented from being hindered by the insertion assisting member, and accordingly, observation or treatment ease can be further improved.

Note that the present invention is not restricted to the above-described embodiments, and that various modifications can be made without departing from the essence of the invention.

## Claims

1. An insertion device comprising:
an insertion unit for assisting insertion into a subject of medical equipment, which is inserted into a subject for performing medical activity, the insertion unit being capable of advancing or retreating relatively as to the medical equipment in the insertion direction into the subject; and
a rotating unit for rotating the insertion unit centered on the insertion axial direction, and generating propulsion at the time of inserting the insertion unit into the subject.

2. The insertion device according to Claim 1, wherein the medical equipment, at the tip of the insertion unit to be inserted into a subject, includes at least either of an observation unit for observing the inside of a subject, or the opening of an insertion tube path into which a treatment tool for performing treatment as to a target portion within a subject is inserted;
and wherein the insertion unit is capable of advancing or retreating relatively in the insertion direction within a subject as to an observation visual field of the observation unit, or the opening of the insertion tube path of the treatment tool.

3. The insertion device according to Claim 2, wherein a propulsion generating unit for generating propulsion along with the rotation is provided at a portion of the insertion unit which can come into contact with an inner wall of a lumen of a subject at the time of inserting the insertion unit into the subject.

4. The insertion device according to Claim 3, wherein the insertion unit includes internal space for inserting the medical equipment therein, and an opening from which can protrude the medical equipment from the tip of the insertion unit.

5. The insertion device according to Claim 3, wherein the insertion unit is inserted into the insertion tube path provided at the medical equipment, and can be protruded from the opening of the tip of the insertion tube path provided in the tip of the medical equipment.

6. The insertion device according to Claim 3, wherein a plurality of the propulsion generating units are provided at the portion of the insertion unit which can come into contact with the inner wall of the lumen of the subject at an equal interval or an arbitrary interval.

7. The insertion device according to Claim 3, wherein the propulsion generating units are consecutively provided at the entire portion which can come into contact with the inner wall of the lumen of the subject.

8. The insertion device according to Claim 6, wherein the propulsion generating units are spiral shaped portions.

9. The insertion device according to Claim 7, wherein the propulsion generating units are spiral shaped portions.

10. The insertion device according to Claim 4, wherein the insertion unit has different flexibility between at the tip side and at the base side.

11. The insertion device according to Claim 5, wherein the insertion unit has different flexibility between at the tip side and at the base side.

12. The insertion device according to Claim 5, wherein the insertion unit comprises a flexible tube body, and a rough wrap-around coil to be disposed at the outer circumferential face side of this tube body.

13. The insertion device according to Claim 5, wherein the insertion unit includes which can come into contact with a subject, and at least a part of surface of the portion has hydrophilicity.

14. The insertion device according to Claim 5, wherein a tip guide member for guiding the tip side of the insertion unit into a deep portion smoothly is provided at the tip side of the insertion unit.

15. The insertion device according to Claim 14, the tip guide member comprising:
a wire member provided at the tip side of the insertion unit; and
a guider provided at the tip side of the wire member.

16. The insertion device according to Claim 15, wherein the flexibility of the wire member is greater than the flexibility of the insertion unit.
